# EUROPEAN PATENT APPLICATION

(11) **EP 1 394 182 A1**
(43) Date of publication of application: **03.03.2004**
(21) Application number: 02090236.7
(22) Date of filing: 08.07.2002
(51) Int. Cl.: C07K 14/82, C12Q 1/68, A61K 39/00, G01N 33/574, C12N 9/02

(54) **Agents and methods for diagnosis and therapy of cancer and cancer risk assessment**

(71) Applicant: Europroteome AG, 16761 Hennigsdorf (DE)
(72) Inventor: Seibert, Volker, Dr., 13187 Berlin (DE); Meuer, Jörn, Dr., 13183 Berlin (DE); Tortola Pérez, Silvia, Dr., 13357 Berlin (DE); Lamer, Stephanie, 13158 Berlin (DE); Rothmann, Kirsten, Dr., 10177 Berlin (DE); Fogeron, Marie-Laure, 13507 Berlin (DE); Buschmann, Thomas, Dr., 13359 Berlin (DE); Ilynia, Tatiana, Dr., 17487 Greifswald (DE); van der Linden, Mark, Dr., 69121 Heidelberg (DE); Heim, Steffen, Dr., 10439 Berlin (DE)
(74) Representative: Ziebig, Marlene, Dr. Dipl.-Chem.

(57) **Abstract**

The invention relates to agents and methods for the diagnosis and therapy of cancer and further to the identification of cancer associated marker. The following tumor marker were identified: Q14259 Enhancer of rudimentary homolog, 000151 PDZ and LIM Domain Protein 1, P55072 Transitional Endoplasmatic Reticulum ATPase, P20142 Gastricsin (pepsinogen C or II), P07492 Beta-2-Glycoprotein I Precursor (apolipoprotein H), P47756 F-Actin Capping Protein Beta subunit, P27824 Calnexin Precursor, P32119 Peroxiredoxin 2, P11021 78 kDa Glucose regulated protein precursor, Q9Y4L1 150 kD Oxygen-regulated Protein Precursor and/or 075493 CA11(downregulated in gastric cancer).

## Description

The invention relates to agents and methods for the diagnosis and therapy of cancer and further to the identification of cancer associated marker. The following tumor marker were identified: Q14259 Enhancer of rudimentary homolog, 000151 PDZ and LIM Domain Protein 1, P55072 Transitional Endoplasmatic Reticulum ATPase, P20142 Gastricsin (pepsinogen C or II), P07492 Beta-2-Glycoprotein I Precursor (apolipoprotein H), P47756 F-Actin Capping Protein Beta subunit, P27824 Calnexin Precursor, P32119 Peroxiredoxin 2, P11021 78 kDa Glucose regulated protein precursor, Q9Y4L1 150 kD Oxygen-regulated Protein Precursor and/or 075493 CA11(downregulated in gastric cancer).

The said proteins provide biological marker for diagnosis and therapy of early and late stage carcinomas and methods of cancer risk assessment. The proteins are members of a protein-superfamily which is connected with cell cycle regulation, cell mobility, oxidative stress response and protein folding, protein translocation and protein degradation.

In spite of improved treatments for certain forms of non-steroid dependent cancer, it is still a leading cause of death in the world. Since the chance for complete remission of cancer is, in most cases, greatly enhanced by early diagnosis, it is very desirable that physicians be able to detect cancers before a substantial tumour develops. Also, in cases where the primary tumour has been substantially removed by surgery or destroyed by other means, it is important that the physician be capable of detecting any trace of cancer in the patient (either in the form of residues of the primary tumour or of secondary tumours caused by metastasis), in order that the physician can prescribe appropriate subsequent treatment, such as chemotherapy.

The quantities of non-steroid dependent cancer cells that must be detected for early diagnosis or following removal or destruction of the primary non-steroid dependent tumour are so small that the physician cannot rely upon physical examination of the cancer site. Moreover, in many cases the cancer site is of course not susceptible to direct visual observation and it is almost always impractical to detect secondary tumours by visual observation, since it is not possible to predict exactly where they are likely to occur. Accordingly, sensitive tests have to rely upon detection of cancer-associated materials, usually proteins, present in body fluids of patients who have, or are about to develop, cancer cells in their bodies. Several diagnostic materials for detection of cancer-associated proteins are available commercially. Tests for alpha-fetoprotein are used to detect primary liver cancer and teratocarcinoma in humans; and carcinoembryonic antigen is used for digestive system cancers, chorionic gonadotropin is employed to detect trophoblast and germ cell cancers; and prostatic acid phosphatase or prostate specific antigen are used to detect prostate carcinoma. These markers are detectable in advanced rather than in early cancer.

Unfortunately, many of the commercially available tests are only applicable to a narrow range of non-steroid dependent cancer types, and therefore these tests suffer not only from the disadvantage that other types of cancer may be missed but also from the disadvantage that the narrow applicability of the tests means that it may be necessary to run multiple tests on a single patient for diagnostic purposes, a procedure which not only increases the expense of the diagnostic testing but also increases the risk that one or other of the tests may give a false positive result. Accordingly, there is a need for a single diagnostic test able to detect the presence of very small amounts of cells of a wide variety of different non-steroid dependent cancers like gastrointenstinal cancer and especially colon carcinome in humans. The ideal marker would be one that is specific and universal. Such a marker may exist if malignant transformation is associated with the expression of a unique gene product in all kinds of transformed cells.

At present, there are no effective presymptomatic clinical signs or biomarkers of susceptibility to non-steroid dependent cancer, making early detection a high priority in medical management of the disease. Efforts to discover prognostic indicators have sought correlations between clinical pathological data and various biochemical parameters. Survival of cancer, whether of the gastrointestinal or colon tissue or another target, is increased through recognition of individuals who are at high risk of a disease, as well as early detection, since current therapeutic strategies for early stage disease have a higher cure rate than for diseases at later stages. For this reason, the identification of molecular markers like proteins of oncogenesis will assist in early diagnosis as well as prognostic monitoring of ongoing disease. Furthermore, if such molecular markers comprise mutations or deletions of genes essential for maintaining normal cellular division, such genes may also be developed as therapeutic agents to treat malignant disease.

Thus, the technical problem underlying the present invention is to provide agents, methods and marker for tumor diagnosis and therapy.

The present invention solves this problem by using Q14259 Enhancer of rudimentary homolog, 000151 PDZ and LIM Domain Protein 1, P55072 Transitional Endolasmatic Reticulum ATPase, P20142 Gastricsin, P07492 Beta-2-Glycoprotein I Precursor, P47756 F-Actin Capping Protein Beta subunit, P27824 Calnexin Precursor, P32119 Peroxiredoxin 2, P11021 78 kDa Glucose regulated protein precursor, Q9Y4L1 150 kD Oxygen-regulated Protein Precursor, 075493 CA11, fragments thereof, nucleic acid sequences encoding said proteins and/or recognition agents against said proteins or said nucleic acid sequences and/or fragments thereof for the manufacture of an agent for diagnosis, prophylactic or therapeutic treatment of non-steroid dependent cancer.

The said proteins are members of a protein-family which is related to cell cycle regulation, cell mobility, oxidative stress response and protein folding, protein translocation and protein degradation. The proteins of the invention that do not occur in nature are isolated. The term isolated as used herein, in the context of proteins, refers to a polypeptide which is unaccompanied by at least some of the material with which it is associated in its natural state. The isolated protein constitutes at least 0.5%, preferably at least 5%, more preferably at least 25% and still more preferably at least 50% by weight of the total protein in a given sample. Most preferably the isolated protein is substantially free of other proteins, lipids, carbohydrates or other materials with which it is naturally associated, and yields a single major band on a non-reducing polyacrylamide gel. Substantially free means that the protein is at least 75%, preferably at least 85%, more preferably at least 95% and most preferably at least 99% free of other proteins, lipids, carbohydrates or other materials with which it is naturally associated.

In the context of the present invention the term recognition agent refers to molecules which interact with proteins or nucleic acid sequences encoding said proteins or fragments thereof. In a embodiment of the present invention the recognition agent is for instance a polyclonal or monoclonal antibody, a lectin, an oligonucleotid or an anti-sense construct. The said proteins, or fragments thereof, may be used to produce polyclonal or monoclonal antibodies, which also may serve as sensitive detection reagents for the presence and accumulation of polypeptides in cultured cells or tissues from living patients; the term patient refers to both humans and animals. The full-length proteins or fragments of the proteins may be used to advantage to generate an array of monoclonal antibodies specific for various epitopes of the proteins, thereby potentially providing even greater sensitivity for detection of the proteins in cells or tissues. Although the recognition agent will conveniently be an antibody, other recognition agents are known or may become available, and can be used in the present invention. For example, antigen binding domain fragments of antibodies, such as Fab fragments, can be used. Also, so-called RNA aptomers may be used. Therefore, unless the context specifically indicates otherwise, the term antibody as used herein is intended to include other recognition agents. Where antibodies are used, they may be polyclonal or monoclonal. Optionally, the antibody can produced by a method so that it recognizes a preselected epitope of said proteins. Polyclonal or monoclonal antibodies immunologically specific for the said proteins may be used in a variety of assays designed to localize and/or quantitate the proteins. Such assays include, but are not limited to: (1) flow cytometric analysis; (2) immunochemical localization of the protein in cultured cells or tissues; and (3) immunoblot analysis; e.g., dot blot, Western blot, of extracts from cells and tissues. Additionally, as described above, such antibodies can be used for the purification of said proteins; e.g, affinity column purification, immunoprecipitation.

Antibody refers in the context of the present invention to a polypeptide substantially encoded by an immunoglobulin gene or immunoglobulin genes, or fragments thereof, which specifically bind and recognize an analyte (antigen). Antibodies exist, e.g., as intact immunoglobulins or as a number of well characterized fragments produced by digestion with various peptidases. The term antibody, as used herein, also includes antibody fragments either produced by the modification of whole antibodies or those synthesized de novo using recombinant DNA methodologies. An antibody specifically binds to or is specifically immunoreactive with a protein when the antibody functions in a binding reaction which is determinative of the presence of the protein in the presence of a heterogeneous population of proteins and other biologics. Thus, under designated immunoassay conditions, the specified antibodies bind preferentially to a particular protein and do not bind in a significant amount to other proteins present in the sample. Specific binding to a protein under such conditions requires an antibody that is selected for its specificity for a particular protein. A variety of immunoassay formats may be used to select antibodies specifically immunoreactive with a particular protein. For example, solid-phase ELISA immunoassays are routinely used to select monoclonal antibodies specifically immunoreactive with a protein.

The present invention also relates to use recognition agents for diagnosis, prophylactic or therapeutic treatment of non-steroid cancer: the use comprises providing recognition agent with means for the recognition of epitopes of any one member of the protein-family on the surface of a tumour cell, whether as part of the complete protein itself or in some degraded form such as in the presentation on the surface of a cell bound to a MHC protein.

In a preferred embodiment of the invention the use of the proteins for diagnosis, prophylactic or therapeutic treatment of non-steroid cancer is characterised by a substance which is capable of stimulation of immune system by activating cytotoxic or helper T-cells which recognise epitopes of any one the proteins in the preparation of a medicament to implement a cell-mediated or humoral immune response against a cell in which any one protein of the protein-family is expressed.

Subject of the invention is further a method for detecting cancer cells in a sample from a patient, the method comprising providing the sample and detecting the level of one or more proteins selected from the group consisting of Q14259 Enhancer of rudimentary homolog, 000151 PDZ and LIM Domain Protein 1, P55072 Transitional Endolasmatic Reticulum ATPase, P20142 Gastricsin (pepsinogen C or II), P07492 Beta-2-Glycoprotein I Precursor (apolipoprotein H), P47756 F-Actin Capping Protein Beta subunit, P27824 Calnexin Precursor, P32119 Peroxiredoxin 2, P11021 78 kDa Glucose regulated protein precursor, Q9Y4L1 150 kD Oxygen-regulated Protein Precursor and/or fragments thereof 075493 CA11(downregulated in gastric cancer) in the sample, and comparing the level of the one or more proteins with a control level that is representative of a level in a normal, cancer-free patient, wherein an modified level of the proteins in the sample compared to the level of the proteins in the control sample indicates the presence of non-steroid dependent cancer in the patient.

The Q14259 Enhancer of rudimentary homolog, 000151 PDZ and LIM Domain Protein 1, P55072 Transitional Endolasmatic Reticulum ATPase, P47756 F-Actin Capping Protein Beta subunit and/or P11021 78 kDa Glucose regulated protein precursor are overexpressed in tumor tissue. The P20142 Gastricsin (pepsinogen C or II), P07492 Beta-2-Glycoprotein I Precursor (apolipoprotein H), P27824 Calnexin Precursor, P32119 Peroxiredoxin 2, P11021 78 kDa Glucose regulated protein precursor, Q9Y4L1 150 kD Oxygen-regulated Protein Precursor and/or 075493 CA11 (downregulate in gastric cancer) are underexpressed in tumor tissue. The present invention is also based on the discovery that the proteins or members of said protein-family are present - over- or underexpressed or up- or down-regulated - in a wide range of malignant non-steroid dependent tumours.

In numerous embodiments of the invention, a method for assessing the presence of non-steroid dependent cancer in a patient will include several steps, including providing a biological sample from the patient, detecting the level of one or more proteins or protein-markers of said protein-family in the sample, and comparing the level of the one or more of said proteins with a control level that is representative of a level in a normal, cancer-free patient. Using such methods, an elevation of marker level in a patient compared to the control level indicates the presence of non-steroid dependent cancer or mesoderm tissue tumour in the patient. The level as used herein can refer to protein level, DNA or RNA level, enzyme activity, the presence of particular isoforms, or any other marker of gene number, expression, or activity. To compare levels of markers means to detect marker levels in two samples and to determine whether the levels are equal or if one or the other is greater. A comparison can be done between quantified levels, allowing statistical comparison between the two values, or in the absence of quantification, for example using qualitative methods of detection such as visual assessment by a human. A control sample refers to a sample of biological material representative of healthy, cancer-free humans. The level of a target in a control sample is desirably typical of the general population of normal, cancer-free humans. This sample can be removed from a patient expressly for use in the methods described in this invention, or can be any biological material representative of normal, cancer-free humans, including cancer-free biological material taken from a human with cancer elsewhere in its body. A control sample can also refer to an established level of a target, representative of the cancer-free population, that has been previously established based on measurements from normal, cancer-free humans.

In a preferred embodiment of present invention the method comprising contacting the sample with a recognition agent for said member of said protein-family selected from the group consisting of Q14259 Enhancer of rudimentary homolog, 000151 PDZ and LIM Domain Protein 1, P55072 Transitional Endolasmatic Reticulum ATPase, P20142 Gastricsin (pepsinogen C or II), P07492 Beta-2-Glycoprotein I Precursor (apolipoprotein H), P47756 F-Actin Capping Protein Beta subunit, P27824 Calnexin Precursor, P32119 Peroxiredoxin 2, P11021 78 kDa Glucose regulated protein precursor, Q9Y4L1 150 kD Oxygen-regulated Protein Precursor 075493 CA11(downregulated in gastric cancer) and/or fragments thereof and/or the nucleic acid sequences encoding said proteins and/or fragments and detecting binding of the recognition agent to any one member of the protein-family or the nucleic acid sequences encoding the proteins in the prepared sample as an indication of the presence of cancer cells in the sample.

Accordingly, an aspect of the embodiment provides a method for the identification of tumour cells, which method comprises the use of a recognition agent, for example an antibody, recognising said proteins to contact a sample of tissues, cells, blood or body product, or samples derived therefrom, and screening for a positive response. The positive response may for example be indicated by an agglutination reaction or by a visualisable change such as a colour change or fluorescence, eg immunostaining, or by a quantitative method such as in the use of radio-immunological methods or enzyme-linked antibody methods.

The method therefore typically includes the steps of (a) obtaining from a patient a tissue sample to be tested for the presence of cancer cells; (b) producing a prepared sample in a sample preparation process; (c) contacting the prepared sample with a recognition agent, such as an antibody or an antisense-construct, that reacts with the any one member or protein of the said protein-family; and (d) detecting binding of the recognition agent to said proteins, if present, in the prepared sample. The human tissue sample can be from for example the bladder, brain, breast, colon, connective tissue, kidney, lung, lymph node, oesophagus, ovary, pancreas, skin, stomach, testis, and uterus or general tissue derived from the mesoderm, especially epithelial tissue.

A practicable sample preparation process includes tissue fixation and production of a thin section. The thin section can then be subjected to immunohistochemical analysis to detect binding of the recognition agent to the members of the said protein-famiy. Preferably, the immunohistochemical analysis includes a conjugated enzyme labelling technique. A preferred thin section preparation method includes formalin fixation and wax embedding. Alternative sample preparation processes include tissue homogenization, and preferably, microsome isolation. When sample preparation includes tissue homogenization, a preferred method for detecting binding of the antibody to the different members of the protein-family is Western blot analysis. Alternatively, an immunoassay can be used to detect binding of the antibody to said proteins. Examples of immunoassays are antibody capture assays, two-antibody sandwich assays, and antigen capture assays. Preferably, the immnunoassay is a solid support-based immunoassay. When Western blot analysis or an immunoassay is used, preferably it includes a conjugated enzyme labelling technique.

In a particularly preferred embodiment of present invention the binding of the recognition agent to any one member of the protein-family in the sample is detected by immunoblotting or immunohistochemical analysis, radioimmunoassay, Western blot analysis mass spectroscopy or enzyme labelling technique. A preferred method for use in the present invention is immunohistochemical analysis. Immunohistochemical analysis advantageously avoids a dilution effect when relatively few cancer cells are in the midst of normal cells. An early step in immunohistochemical analysis is tissue fixation, which preserves proteins in place within cells. This prevents substantial mixing of proteins from different cells. As a result, surrounding normal cells do not diminish the detectability of the member of the protein-family-containing cancer cells. This is in contrast to assay methods that involve tissue homogenization. Upon tissue homogenization, said proteins from cancer cells are mixed with proteins from any surrounding normal cells present in the tissue sample. The concentration of said proteins are thus reduced in the prepared sample, and it can fall below detectable limits. Immunohistochemical analysis has at least three other advantages. First, it requires less tissue than is required by alternative methods such as Western blot analysis or immunoassay. Second, it provides information on the intracellular localization and distribution of immunoreactive material. Third, information on cell morphology can be obtained from the same thin section used to test for the presence of said proteins. Preferably, when immunohistochemical analysis is employed in the practice of this invention, several thin sections from each tissue sample are prepared and analysed. This increases the chances of finding small tumours. Another preferred antibody method for use in the present invention is Western blot analysis, ie sodium dodecyl sulfate-polyacrylamide gel electrophoresis (SDS-PAGE), followed by immunoblotting. Sample preparation for Western blot analysis includes tissue homogenization, and optionally isolation of microsomes. Western blot analysis has the advantage of detecting immunoreactivity on proteins that have been separated with high resolution, according to apparent molecular weight. Inmunoassays such as antibody capture assays, two-antibody sandwich assays, and antigen capture assays can also be used in the present invention. Sample preparation for immunoassays includes tissue homogenization, and optionally isolation of microsomes. Immunoassays have the advantage of enabling large numbers of samples to be tested relatively quickly, and they offer quantitative precision. Principles and practice of immunohistochemistry, Western blot analysis, and immunoassays are well known. One of ordinary skill in the art can select suitable protocols and carry out immunohistochemical analysis. Western blot analysis, or an immunoassay, in the practice of the present invention.

In a preferred embodiment of the invention said proteins are detected by determining the copy number of nucleic acids encoding for the proteins or fragments thereof in the sample. Detecting a level of said marker proteins refers also to determining the expression level of a gene or genes encoding a target polypeptide or protein. The copy number of a gene can be measured in multiple ways known to those of skill in the art, including, but not limited to, Comparative Genomic Hybridization (CGH) and quantitative DNA amplification (e.g., quantitative PCR) . Gene expression can be monitored in a variety of ways, including by detecting mRNA levels, protein levels, or protein activity, any of which can be measured using standard techniques. Detection can involve quantification of the level of (e.g., gDNA, cDNA, mRNA, protein, or enzyme activity), or, alternatively, can be a qualitative assessment of the level of a target, in particular in comparison with a control level.

A preferred embodiment method for detecting the presence of nucleic acids encoding for said proteins in the sample, comprising the steps of:
a.) providing a nucleic acid from a biological material of the sample,
b.) contacting the nucleic acid with a nucleic acid segment of continuous sequence that hybridizes under stringent conditions to a contiguous sequence of any one of said proteins under conditions effective to allow hybridization of substantially complementary nucleic acids; and
c.) detecting the hybridized complementary nucleic acids thus formed.

Persons skilled in the art will appreciate that nucleotide sequences having sufficient homology to be considered substantially the same are often identified by hybridization to one another under appropriate hybridization conditions.

An another preferred method for detecting the presence of nucleic acids encoding for any one of the proteins of of said protein-family in the sample, comprising the steps of:
a.) obtaining nucleic acids from the sample suspected of containing any one of said proteins,
b.) isolating nucleic acids from the sample,
c.) contacting said nucleic acids with a nucleic acid segment of continuous sequence that hybridizes under stringent conditions to a contiguous sequence of any one of said proteins under conditions effective to allow hybridization of substantially complementary nucleic acids, and
d.) detecting the hybridized complementary nucleic acids thus formed.

In a preferred method the sample nucleic acids contacted are located within a cell, wherein the sample nucleic acids are DNA or RNA.

The present invention also relates to a method of obtaining drugs of potential use in cancer therapy, which comprises screening for or selecting a substance which is susceptible to specific metabolism by any one member of the protein-family, and using that substance as a basis for a non-toxic moiety which can be converted by the metabolism of any one member of the protein-family into a toxic one, which kills or inhibits a tumour cell expressing one of any one member of the protein-family or makes it more susceptible to other agents. This other aspect of the invention lies in the presence of said proteins selectively in tumours which provides a mechanism for the selective targeting of anti-cancer drugs based on metabolism in tumours. Drugs can be designed for, or screened for, specific metabolism by said proteins in tumours whereby this metabolism converts a non-toxic moiety into a toxic one.

The present invention also relates to a method of obtaining drugs of potential use in cancer therapy, which comprises screening for or selecting a substance which is susceptible to specific metabolism by any one member of the protein-family, and using that substance as a basis for a non-toxic moiety which can be converted by the metabolism of any one member of the protein-family into a toxic one, which kills or inhibits a tumour cell expressing of any one member of the protein-family or makes it more susceptible to other agents. This another aspect of the invention provides for the targeting of cytotoxic drugs or other therapeutic agents, or the targeting of imaging agents, by virtue of their recognition of epitopes of said proteins on the surface of a tumour cell, whether as part of the complete said proteins itself or in some degraded form such as in the presentation on the surface of a cell bound to a MHC protein. The present invention also relates to use the proteins, the recognition agents, nucleic acids and/or fragments thereof for diagnosis, prophylactic or therapeutic treatment of cancer.

The invention also relates also to a method of treatment of non-steroid dependent cancer, characterised by a modification of the level of the proteins, the recognition agents and/or said nucleic acids and/or fragments thereof.

In a preferred embodiment the method of treatment of cancer is characterised by a reduction of levels of Q14259 Enhancer of rudimentary homolog, 000151 PDZ and LIM Domain Protein 1, P55072 Transitional Endolasmatic Reticulum ATPase, P47756 F-Actin Capping Protein Beta subunit and/or P11021 78 kDa Glucose regulated protein and/or fragments thereof.

In a preferred embodiment of present invention the a method of treatment of non-steroid dependent cancer is characterised by the reduction of levels of Q14259 Enhancer of rudimentary homolog, 000151 PDZ and LIM Domain Protein 1, P55072 Transitional Endolasmatic Reticulum ATPase, P47756 F-Actin Capping Protein Beta subunit and/or P11021 78 kDa Glucose regulated protein precursor in tumour cells in the preparation of a medicament, wherein the substance comprising an inhibitor or means for producing antisense RNA to decrease the synthesis of Q14259 Enhancer of rudimentary homolog, 000151 PDZ and LIM Domain Protein 1, P55072 Transitional Endolasmatic Reticulum ATPase, P47756 F-Actin Capping Protein Beta subunit and/or P11021 78 kDa Glucose regulated protein.

In a preferred embodiment of the method a promoter of Q14259 Enhancer of rudimentary homolog, 000151 PDZ and LIM Domain Protein 1, P55072 Transitional Endolasmatic Reticulum ATPase, P47756 F-Actin Capping Protein Beta subunit and/or P11021 78 kDa Glucose regulated protein is down-regulated.

In a preferred embodiment the method of treatment of cancer is characterised by a raising of levels of P20142 Gastricsin, P07492 Beta-2-Glycoprotein I Precursor, P27824 Calnexin Precursor, P32119 Peroxiredoxin 2, P11021 78 kDa Glucose regulated protein precursor, Q9Y4L1 150 kD Oxygen-regulated Protein Precursor and/or 075493 CA11.

In another preferred embodiment of the invention, the method of treatment is characterised by the raising of levels of P20142 Gastricsin (pepsinogen C or II), P07492 Beta-2-Glycoprotein I Precursor (apolipoprotein H), P27824 Calnexin Precursor, P32119 Peroxiredoxin 2, P11021 78 kDa Glucose regulated protein precursor, Q9Y4L1 150 kD Oxygen-regulated Protein Precursor and/or 075493 CA11(downregulated in gastric cancer) in tumour cells in the preparation of a medicament, wherein the substance comprising a stimulator, copy genes or promoter to increase the synthesis of P20142 Gastricsin (pepsinogen C or II), P07492 Beta-2-Glycoprotein I Precursor (apolipoprotein H), P27824 Calnexin Precursor, P32119 Peroxiredoxin 2, P11021 78 kDa Glucose regulated protein precursor, Q9Y4L1 150 kD Oxygen-regulated Protein Precursor and/or 075493 CA11 (downregulate in gastric cancer).

In a preferred embodiment the method of treatment of tumour is characterised by up-regulation of promoters of P20142 Gastricsin (pepsinogen C or II), P07492 Beta-2-Glycoprotein I Precursor (apolipoprotein H), P27824 Calnexin Precursor, P32119 Peroxiredoxin 2, P11021 78 kDa Glucose regulated protein precursor, Q9Y4L1 150 kD Oxygen-regulated Protein Precursor and/or 075493 CA11(downregulated in gastric cancer).

The invention also relates to a kit for detecting of cancer cells in a sample comprising at least one of said proteins, said recognition agents or said nucleic acids or nucleic acid sequences.

The discovery that the proteins or members of said protein-family are detectable in a wide range of human non-steroid dependent cancers of different histogenetic types gives rise to diagnostic methods for detecting tumours based on these proteins or the genes of the proteins as marker, and to the possibility of tumour therapies involving the proteins or their antibodies or genes.

From the foregoing discussion, it can be seen that said protein and antibodies thereto and nucleic sequences which encoded the proteins can be used in many ways for diagnosis and prognosis of human neoplastic diseases of mesoderm tissue, preferably gastrointestinal tumor. However, one person skilled in the art will appreciate that these tools will also be useful in animal and cultured cell experimentation with respect to various carcinomas. They can be used to monitor the effectiveness of potential anti-cancer agents on cellular proliferation in vitro, and/or to assess the development of neoplasms or other malignant diseases in animal model systems.

The members or proteins of the said protein-family, antibodies thereto or antisense-sequences, provide a much-needed molecular marker for oncogenesis that will assist in early diagnosis and prognostic monitoring of malignant disease, particularly gastrointestinal cancer. The gens which encoded proteins also will be useful in gene replacement therapy or for the development of other therapeutic agents to treat various forms of malignant disease of mesoderm tissue.

The following examples are provided to describe the invention in further detail. These examples are intended to illustrate and not to limit the invention.

### Example

### Detection and Characterization of the protein-markers

### First dimension - Isoelectrofocusing (IEF)

### Sample denaturation

Material: human tumor tissue: The cell pellets are denaturated with a volume of denaturation solution which has to be adjusted depending of the pellet size (ratio pellet to buffer ∼ 1 to 4). After addition of the denaturation solution the samples are vortexed, sonicated three times 10 seconds using the Sonoplus (Bandelin) and centrifuged for 10 minutes at 4°C with maximal speed. They can then be stored at -20°C.

### Protein concentration evaluation

The protein concentration is determined according to the Bradford assay in accordance with the instructions of Bio-Rad and as described in the attachment.

### In-gel sample rehydration

100µg of protein samples (analytical 2D-PAGE) or 3mg of protein samples (preparative 2D-PAGE), solubilized in 300µl when working with IPG strips from Bio-Rad (or 350µl when working with the strips from Amersham Biosciences) of the above mentioned solution, are pipetted in the focusing tray. After removing the protection film, the IPG strips are then positioned such that the gel of the strip is in contact with the sample (up side down). The gel and the sample are covered with about 2ml low viscosity oil (Mineral oil from Bio-Rad, Cat.No.163-2129) to avoid evaporation. The strips are rehydrated for 8 hours, a low voltage of 50V being applied.

### Focusing

The strips are focused at 20°C under an increasing voltage from 300V to 3500V during 3 hours, followed by 3 additional hours at 3500V [the voltage can also be linearly increased from 300V to 3500V for 8 hours] , the intensity should be 50µA per strip (2mA max. in total). Whereupon the voltage is increased to 10000V until achieving a volthourproduct of 80-100kVh.

After running, the strips can be frozen in glass tubes at -20°C for several weeks, or used immediately for the second dimension.

### Second dimension - SDS-PAGE

### Equilibration of the strips

After the first dimension run the strips are equilibrated in order to resolubilise the proteins reduce-S-S- bonds. Each strip is equilibrated twice during 15 minutes (with shaking) with 10ml of equilibration solution. For the first equilibration step, dithiotreitol (DTT) is added to the above mentioned solution at a concentration of 10mg/ml [to ensure that any reformed disulphide bridges are reduced], whereas iodoacetamide (IAA) at a concentration of 48mg/ml is added to the same solution for the following equilibration step [to alkylate the proteins and to react with any reduced DTT]. After the equilibration, rinse the IPG strips with deionized water for a second and place them on a piece of filter paper at one edge for a few minutes to drain off excess equilibration buffer.

### Running SDS gels

When working with the **Ettan Dalt II** (Amersham) : Use the Buffer Kit from Amersham (Cat.No.17-6002-36) in accordance to the instructions. Place the strip on the top of a precast gel 12.5 (Cat.No.17-6002-36), as well as a wick (on the right end of the strip) on which protein molecular weight standards (Amerham, Cat.No.RPN5800) are loaded. For the preparation of these standards, mix the markers solution with the loading buffer (1:4), and denaturate for 4 min. at 95°C. Pipette the sealing solution on the top of the gel and press carefully the strip with a spatula to achieve complete contact.

The migration conditions are the following : 2,5 W/gel for 30 minutes and 19 W/gel (170W maximum) for 5 hours until the bromophenol blue tracking dye is going out of the gel.

When working with the **MultiCell** or the **Criterion Dodeca Cell** (Bio-Rad): seal hot 0,5% agarose solution on top of an SDS gel, and place quickly the strip on top of this gel. Carefully press the strip with a spatula onto the surface of the SDS gel to achieve complete contact. Allow the agarose to solidify for at least 5 minutes. Molecular weight markers can also be used. The migration conditions are the following : 25 V and 40 mA/gel during 1 hour, and then 600 V and 40 mA/gel during 5 or 6 hours, until the bromophenol blue tracking dye has migrated off the lower end of the gel. Electrophoresis buffer 1X has to be prepared.

### Silver Staining

The following steps are realized at room temperature with the Hoefer Processor Plus :
- Fixation : the gels are fixed in 50% methanol / 5% acetic acid in water overnight.
- The gels are washed for 10 minutes with 50% methanol in water, and additionally for 10 minutes with water to remove the remaining acid.
- Sensitizing : the gels are sensitized by a 1 minute incubation in 0,02% sodium thiosulfate.
- The gels are rinsed twice with distilled water for 1 minute.
- Staining : the gels are incubated in chilled 0,1% silver nitrate solution for 20 minutes.
- The gels are rinsed twice with distilled water for 1 minute.
- Development : the gels are developed in developing solution.
- Stop of the reaction : with a solution of 5% acetic acid in water.

Silver-stained gels are stored in a solution of 1% acetic acid at 4°C until being analyzed.

### Isolation of total RNA

- to get good RNA out of samples it is necessary to wear gloves all the time and use only RNAse- and DNAse free tips and tubes or to autoclave them
- we use the Qiagen RNeasy Mini Kit Cat no.74 106
   1. before use : add β-Mercaptoethanol to buffer RLT (1µl β-ME/1ml buffer)
      add 96% ethanol to buffer RPE as indicated on the bottle
   2. add 350µl or 600µl buffer RLT (depending on the pellet,larger pellets might even need more RLT buffer) to lyse the cells
   3. homogenize the sample with the rotor stator (30s) or with the sonicator (10s
   4. add the same volume of 70% ethanol to the sample and mix by pipetting ( do not centrifuge and vortex)
   5. apply 700µl of this mix to an Rneasy mini spin column and close it if the volume exceeds 700µl load aliquots onto the column
   6. centrifuge for 15s at 10.000rpm (sometimes it is necessary to centrifuge higher or longer - it depends on the sample viscosity and the possibility to get it trough the column)
   7. discard the flow-trough and reuse the collection tube
   8. add 700µl buffer RW1 (in Kit)
   9. centrifuge for 15s at 10.000rpm
   10. discard the flow-trough and the collection tube and transfer the column in a new collection tube
   11. add 500µl buffer RPE (in Kit)
   12. centrifuge for 15s at 10.000rpm/ discard the flow-trough
   13. add another 500µl buffer RPE
   14. centrifuge for 2min at high speed to get rid of the buffer
   15. to elute transfer the column to a new 1,5ml collecting tube and pipet 30-50µl Rnase free water (in Kit) directly on the silica- gel membrane
   16. centrifuge for 1min at 10.000rpm
   17. repeat step 15.and 16. in the same tube
   18. close

### Agilent Direct-Label cDNA Synthesis Kit Protocol

We use the Agilent direct label Kit (Cat no. G2555A) Also we need Cyanine 3-dCTP and Cyanine 5-dCTP from Perkin Elmer (NEL 576/ NEL 577)
- Labeling of total RNA requires 20µg per labeling reaction - the amount of RNA is determined with the bioanalyzer (at least 3 dilutions)
- Prepare one tube for cyanine 3 and one for cyanine 5 for each sample (this is because of the dye-swop: there a two arrays on one chip. Each is hybridizied with a Cy 3 and Cy5 labeled sample. On array A01 ( the one next to the barcode) we always put NT labelled with Cy3 plus TU labeled with Cy5. On array A02 the TU sample is labelled with Cy3 and is incubated together with the Cy5 labeled NT sample.)
- Pipet the 20µg of RNA to each reaction tube
- Add 2µl DNA Primer (Agilent Kit) and bring the total sample volume to 50µl in nuclease free water
- Incubate tube at 70°C for 10 minutes
- Place reaction tube on ice for 5 minutes
- During these 15 minutes incubation time prepare the master mix (all in Agilent Kit)
- Master Mix for 1 Chip/ 4 labeling reactions :
   57,4 µl nuclease - free water
   82 µl 5x First strand buffer
   41 µl 0,1 M DTT
   4,1 µl dNTP Mix
   2,05 µl 5mM dCTP
   8,2 µl MMLV-RT
- store the Master Mix on ice until use
- add to the reaction tubes 2,5µl of either cyanine 3-dCTP or cyanine 5-dCTP
- pipet 47,5 µl of the Master Mix into each sample tube
- incubate cDNA synthesis reaction at 42°C for 60 minutes
- move the reaction to heating block at 70°C for 10 minutes
- place reaction tube on ice for 5 minutes
- add 2,0 µl RNAse IA (Agilent Kit) to each tube and incubate at room temperature for 30 minutes

### Purification of labeled cDNA

- We use the QIAquick PCR Purification Kit from Qiagen (Cat no. 28104)
- Add 5 volumes of buffer PB (in Kit) to each reaction tube (we have 100µl in the tubes, so we add 500µl buffer)
- Apply the sample to the Qiaquick column which is placed in a 2ml collection tube
- Centrifuge for 60s at 13000 rpm
- Discard the flow- through and reuse the collection tube
- Add 750µl of 35% guanidine hydrochloride (see solutions) and centrifuge for 60s at 13000rpm
- Discard the flow-through and reuse the collection tube
- Wash the column with 750µl Qiagen's buffer PE (add ethanol to the buffer before use; its indicated on the bottle) and centrifuge for 60s at 13000rpm
- Discard the flow-trough and centrifuge again for 60s at high speed to remove any residual ethanol
- Place the column into a clean collection tube
- Add 30µl Qiagen buffer EB to the center of the column , wait for 1 minute, then centrifuge for 60s at 13000rpm
- Repeat this step with additional 30µl of buffer EB and elute into the same tube, so that the final volume should be approximately 60µl
- Combine the appropriate cyanine 3- cDNA and cyanine 5-cDNA in one tube (that means non-tumour 3 and tumour 5 in one tube and non-tumour 5 and tumour 3 in one tube)
- Dry the solution under vaccum in rotary dessicator (Speed Vac) approximately 60 minutes

### Preparation of the Hybridization solution

- Resuspend, by gently pipetting, the cyanine-3/cyanine-5 labeled cDNA in 7,5µl nuclease- free water
- Store on ice
- Add to the tubes 2,5µl Deposition Control Target (Operon; Cat no. SP300)
   2,5µl Human Cot-1 DNA (Invitrogen; Cat no.15279-011)
   12,5µl Deposition Hybridization buffer (Agilent Kit)
- incubate at 98°C for 2 minutes
- centrifuge 60s at high speed and then leave the solution at room temperature until use

### Hybridization of labeled cDNA to slides

This step is very important and you have to be very careful, because the microarray surface is very delicate. Any touching will destroy the array.
- Place the slide on the microarray slide template (included with the kit) with the barcode facing down
- Pipet 25µl of the prepared Hybridization mixture onto the left side from the active surface without any air bubbles
- Place a clean coverslip onto the same side and slowly lower the coverslip to allow the hybridization mixture to fill the whole surface of the array (without air bubbles)
- Place the slide into the hybridisation chamber base (the chamber is in the third drawer next to the sink)
- Pipet in the corners of the base approximately 15µl nuclease-free water
- Place the cover on the top of the base and close the chamber (screw tight)
- Incubate overnight (approximately 17 hours) in a 65°C waterbath

### Washing slides

- Prepare wash solution 1 and 2 (see solution)
- Pass solution through a 0,2µm sterile filtration unit to avoid dust
- Store the solution at room temperature
- Prepare 4 staining dishes :
   one with solution 1, two with solution 2 and one with water
   And a beaker (500ml) with solution 1
- Remove the hybridization chamber from the waterbath and dry it before loosening the screws
- Remove coverslips by gently dipping the slide in the glass with wash solution 1
- Place the slide in the staining dish with solution 1 , put them on a magnetic stirrer and stir for 10 minutes (medium)
- Transfer the slides into the first dish with wash solution 2 and submerse the arrays, to get rid of the wash solution one
- transfer the slides into the second dish with wash solution 2 and stir for 5 minutes (medium)
- dip the slides into the dish with water
- carry the dish with the slides to the centrifuge and quickly transfer the slides into black plastic racks
- immediately put the racks (with the slides) in the centrifuge and start - everything must be prepared before the drying procedure is started
- centrifuge for 2 minutes at 400g (2000rpm)
- transfer the slide in the scanning box (can be found in the cupboard over the Bioanalyzer) and put it into the carousel - do not put it into the home position which is marked with "H"
- put the carousel in the scanner
   (click the bottom "Agilent scanner" and click "browse " in the . scanner window and Save the data under H/public/scanner... make a new folder for every scan with the name of the sample and the date)
- After it is turned on the scanner needs about 20-30 minutes to completely warm up. Do not scan until the scanner is ready.
- scan (press "scan" if he is ready, who have to say in what directory he should save the data)
- store the slides in a polypropylene slide box in the dark at room temperature

### Quality control (RNA)

- For the quality control we use the Agilent Bioanalyzer and the Agilent RNA 6000 Nano Assay Lab chip (Nr. 5064-8229); store at 4°C in the fridge of the RNA lab
- Additionally we need RNA 6000 ladder from Ambion (Cat. No 7152); store at -20°C

### Preparing the gel dye mix

- Place 400µl RNA gel matrix (red top in the Kit) into the top of the spin filter
- Centrifuge them 10min at 5000rpm
- Place 130µl of the filtered RNA gel matrix in a RNAse free tube and add 2µl of RNA dye concentrate (blue top)
- The rest you can store in the fridge and use it later in the same way with the dye
- Vortex the mix and aliquot into 30µl aliquots
- Protect the gel dye mix from light and store the mix at 4°C
- Use it within one week

### Preparing the ladder

- Aliquot the ladder into 5µl aliquots and heat one of them for 2min at 70-80°C
   (this heated ladder you can use only for two weeks and then you have to heat another tube)
- Store the ladder aliquots at 4°C

### Chip Priming station

- The chip priming station has two positions :
   the base plate has to be on position C
   The syringe- clip at the topmost position
(there is a picture of this in the manual from Agilent in the first drawer under the Bioanalyzer)

### Loading the Chip

- Before use warm all things to room temperature
- Pipette 9µl of gel- dye Mix at the bottom of the well marked with the black G
- Make sure that the plunger is at 1ml
- Press the plunger until it is held by the syringe clip
- Wait for exactly 30s and then pull back the plunger to the 1ml portion
- Open the Chip Priming station
- Pipette 9µl of gel-dye mix in the other wells marked G
- Pipette in all other wells 5µl of the RNA 6000 Marker (green cap in Kit)
- Pipette 1µl of the ladder at the bottom of the well with the ladder sign
- Pipette 1µl of each sample you have into each of the 12 sample wells
- Place the chip in the adapter of the vortex mixer and vortex for exactly 1 minute
- Place the chip in the Bioanalyzer and start the run within 5 minutes

### Running the RNA Assay

- Select the assay from the Assay menue (Assay - RNA - Eukaryote total RNA Nano)
- Press start
- When the start box appears you can enter the number of samples
- Press start again
- Now you can complete the sample list with the name and the dilution in a table and press ok
- The chip runs
- Data will be saved automatically in a folder only for the bioanalyzer

### Cleaning after an Assay

- Open the bioanalyzer and remove the chip
- Place the cleaning chip filled with 350µl RNAse free water into the bioanalyzer
- Close the lid for about 10 seconds
- Open it and remove the cleaning chip and wait another 10 seconds for the water to evaporate
- Close the lid

### Checking your RNA results

Major features of a successful ladder run are:
- 6 RNA peaks
- 1 Marker peak (that's the first one)
- all 7 peaks are well resolved
- if a yellow sign over the ladder line appears, than you have to change the highest setpoint in the settings from 2.0 to 0.5 and press apply
- major features for a successful total RNA run are:
   - 2 ribosomal peaks (with a successful sample preparation at time 39-42 s and 45-50 s)
   - 1 marker peak (at the same position like in the ladder)
- in "results" the bioanalyzer shows the amount of RNA
   - **pH range :**: 3-6 **Search done on 10 patients**
   - **Samples :**: P01.0103_SNCX0000001Z = MD5nt
   P01.0104_STCX0000002Z = MD5tu
   P01.0105_SNCX0000003Z = MD9nt
   P010106_STCX0000004Z = MD9tu
   P01.0107_SNCX0000006Z = MD11nt
   P01.0108_STCX0000007Z = MD11tu
   P01.0112_SNCX0000011Y = MD13nt
   P01.0113_STCX0000012Y = MD13tu
   P010114_SNCX0000013X = MD17nt
   P01.0115_STCX0000014Y= MD17tu
   P010116_SNCX0000015Y = MD21 nt
   P01.0117_STCX0000016Y = MD21tu
   P01.0118_SNCX0000017Y = MD22nt
   P01.0119_STCX0000018Y = MD22tu
   P01.0143_MD51nt
   P01.0144_MD51tu
   P01.0145_MD56nt
   P01.0146_MD56tu
   P01.0147_MD60nt
   P01.0148_MD60tu
   - **ms :**: protein identified by mass spectrometry
   - **ni:**: not identified by mass spectrometry
   - **no comment :**: protein not analysed by mass spectrometry
   - **Expression +**: up-regulated protein
   - **Expression -**: down-regulated protein
   - **T:**: expressed only in tumor
   - **N:**: expressed only in normal

| Reproducibility | Name | Swissprot | NCBI | Expression | Comments |
|---|---|---|---|---|---|
| 7 | Gastricsin Precursor | P20142 | | - | |
| 6 | Cathepsin E Precursor / Gastricin precursor (Pepsonogen C)* | P14091/ P020142* | | - | both proteins are cleary confirmed with PSD, only 1 or two peptides are matching in PMF |
| 6 | down regulated in gastric cancer | | 9665240 | - | same protein than CA1, also observed in pH5-8 |
| 6 | CA11 protein | Q9NS71 | | - | same protein than down-regulated protein in gastric cancer |
| 6 | Spasmolytic Polypeptide Precursor | Q03403 | | - | |
| 5 | Nucleophosmin | P06748 | | + | |
| 5 | Gastricsin Precursor | P20142 | | - | |
| 5 | Epithelial-Cadherin Precursor | P12830 | | - | |
| 5 | Cathepsin E precursor | P14091 | | - | |
| 5 | not identified | | | - | |
| 5 | Hemoglobin Beta Chain | P02023 | | - | |
| 5 | Myosin Light Chain Alkali, non muscle isoform / Myosin Light Chain Alkali, smooth-muscle* | P16475 / P24572* | | + | matching with same peptides |
| 4 | Tropomyosin, Fibroblast non-muscle type* / Tropomyosin Beta Chain, Fibroblast and Epithelial | P07226* / P06468 | | + | more than one Tropomyosin in DB (with equal matching peptides) |
| 4 | ATP Synthase Beta Chain, mitochondrial precursor | P06576 | | - | |
| 4 | not identified | | | - | |
| 4 | not identified | | | - | |
| 4 | Nucleobindin 1 precursor | Q02818 | | - | |
| 4 | ATP Synthase Beta Chain | P06576 | | - | |
| 4 | Myosin regulatory light chain 2, smooth muscle isoform / Myosin regulatory light chain 2, nonsarcomeric^{*} | P24844 / P19105* | | + | matching with same peptides |
| 4 | Protein Disulfide Isomerase Precursor | P07237 | | - | |
| 3 | not identified | | | + | |
| 3 | Vimentin | P08670 | | + | |
| 3 | Vimentin | P08670 | | + | |
| 3 | not identified | | | - | |
| 3 | Complement Component 1, Q Subcomponent Binding Protein | Q07021 | | - | |
| 3 | ATP Synthase Beta Chain, mitochondrial precursor | P06576 | | - | |
| 3 | Protein Disulfide Isomerase Precursor | P07237 | | | |
| 3 | Cytokeratin 20 | P35900 | | | |
| 3 | (XM_006710) ATP synthase, H+ transporting, mitochondrial F1 complex / ATP syntase beta chain, mitochondrial precursor* | P06576* | 18601447 | - | |
| 3 | (BC007716) unknown / (AF058954) GTP-specific succinyl-CoA synthetase beta subunit* | | 14043451/37 | - | |
| 3 | ATP Synthase Beta Chain | P06576 | | - | |
| 3 | Triacylglycerol Lipase, gastric precursor / ATP Synthase Beta Chain* | P07098 / P06576* | | - | |
| 3 | Pepsin A precursor | P00790 | | - | |
| 3 | not identified | | | + | |
| 3 | Calgranulin B | P06702 | | + | |
| 3 | not identified | | | - | |
| 3 | Cathepsin E Precursor | P14091 | | - | |
| 3 | ATP Synthase D Chain | O75947 | | - | |
| 2 | not identified | | | + | |
| 2 | Transional Endoplasmic Reticulum ATPase | P55072 | | + | |
| 2 | Enhancer of rudimentary homolog | Q14259 | | + | |
| 2 | F-Actin Capping Protein Beta subunit | P47756 | | + | |
| 2 | Vimentin | P08670 | | + | |
| 2 | Vimentin | P08670 | | + | |
| 2 | Alpha-Soluble NSF Attachment Protein | P54920 | | + | |
| 2 | Cytokeratin 7 Cytokeratin 8 | P08729 P05787 | | + | |
| 2 | not identified | | | - | |
| 2 | Alpha-1 B-Glycoprotein | P04217 | | - | PSD only low intensity |
| 2 | Calnexin Precursor | P27824 | | - | |
| 2 | Cytokeratin 8 | P05787 | | - | |
| 2 | Peroxiredoxin 2 | P32119 | | - | |
| 2 | Actin, Cytoplasmic 1/ Actin, Cytoplasmic 2* | P02570/P02571* * | | - | |
| 2 | Actin, Cytoplasmic 1/ Actin, Cytoplasmic 2* | P02570 / P02571 * | | - | |
| 2 | Protein Disulfide Isomerase Precursor | P07237 | | - | |
| 2 | Cytochrom B5 | P00167 | | - | |
| 2 | Pepsin A precursor | P00790 | | - | |
| 2 | (X05606 beta subunit / ATP Synthase Beta Chain* | P06576* | 28931 | - | |
| 2 | Triacylglycerol Lipase, gastric precursor | P07098 | | - | |
| 2 | not identified | | | | |
| 2 | Cytokeratin 19, Actin, Cytoplasmic 1*, Actin, Cytoplasmic 2** | P08727, P02570*, P02571*\| | | + | |
| 2 | not identified | | | + | |
| 2 | Actin, Cytoplasmic 1/ Actin, Cytoplasmic 2* | P02570 / P02571* | | + | |
| 2 | not identified | | | + | |
| 2 | (BC016277) unknown / (AF117615) heme-binding protein* | | 9622095 / 770 | + | both proteins have similar sequences |
| 2 | Tropomyosin, Fibroblast non-muscle type | P07226 | | + | |
| 2 | Collagen alpha 1 (VI) Chain precursor | P12109 | | + | |
| 2 | Cathepsin B Precursor | P07858 | | + | |
| 2 | not identified | | | - | |
| 2 | Cytokeratin 8 8 | P05787 | | - | |
| 2 | not identified | | | - | |
| 2 | Cathepsin E Precursor | P14091 | | - | |
| 2 | Aldehyde Dehydrogenase | P05091 | | - | |
| 2 | Immunoglobulin J Chain | P01591 | | - | |
| 1 | Tropomyosin, Fibroblast non-muscle type | P07226 | | + | |
| 1 | Cathepsin D Precursor | P07339 | | + | |
| 1 | ATP Synthase Beta Chain, mitochondrial precursor | P06576 | | - | |
| 1 . | Protein Disulfide Isomerase Precursor | P07237 | | + | underexpressed for three other patients |
| 1 | Heat Shock Cognate 71 kDa Protein | P11142 | | + | |
| 1 | Proteasome activator hPA28 subunit beta | | 4506237 | + | |
| 1 | Vimentin | P08670 | | + | |

- **pH range :**: 5-8 **Search done on 10 patients**
- **Samples :**: P01.0075_SNCX0000001Z = MD5nt
P01.0076_STCX0000002Z = MD5tu
P01.0077_SNCX0000003Z = MD9nt
P01.0078_STCX0000004Z = MD9tu
P01.0079_SNCX0000006Z = MD11 nt
P01.0080_STCX0000007Z = MD11tu
P01.0083_SNCX0000011Y = MD13nt
P01.0084_STCX0000012Y = MD13tu
P01.0085_SNCX0000013X = MD17nt
P01.0124_STCX0000014Y = MD17tu
P01.0087_SNCX0000015Y = MD21nt
P01.0088_STCX0000016Y = MD21tu
P01.0089_SNCX0000017Y = MD22nt
P01.0090_STCX0000018Y = MD22tu
P01.0149_MD51nt nt
P01.0150_MD51tu tu
P01.0151_MD56nt
P01.0152_MD56tu
P01.0153_MD60nt
P01.0154_MD60tu
- **ms :**: protein identified by mass spectrometry
- **ni:**: not identified by mass spectrometry
- **no comment :**: protein not analysed by mass spectrometry
- **Expression +**: up-regulated protein
- **Expression -**: down-regulated protein
- **T:**: expressed only in tumor
- **N:**: expressed only in normal

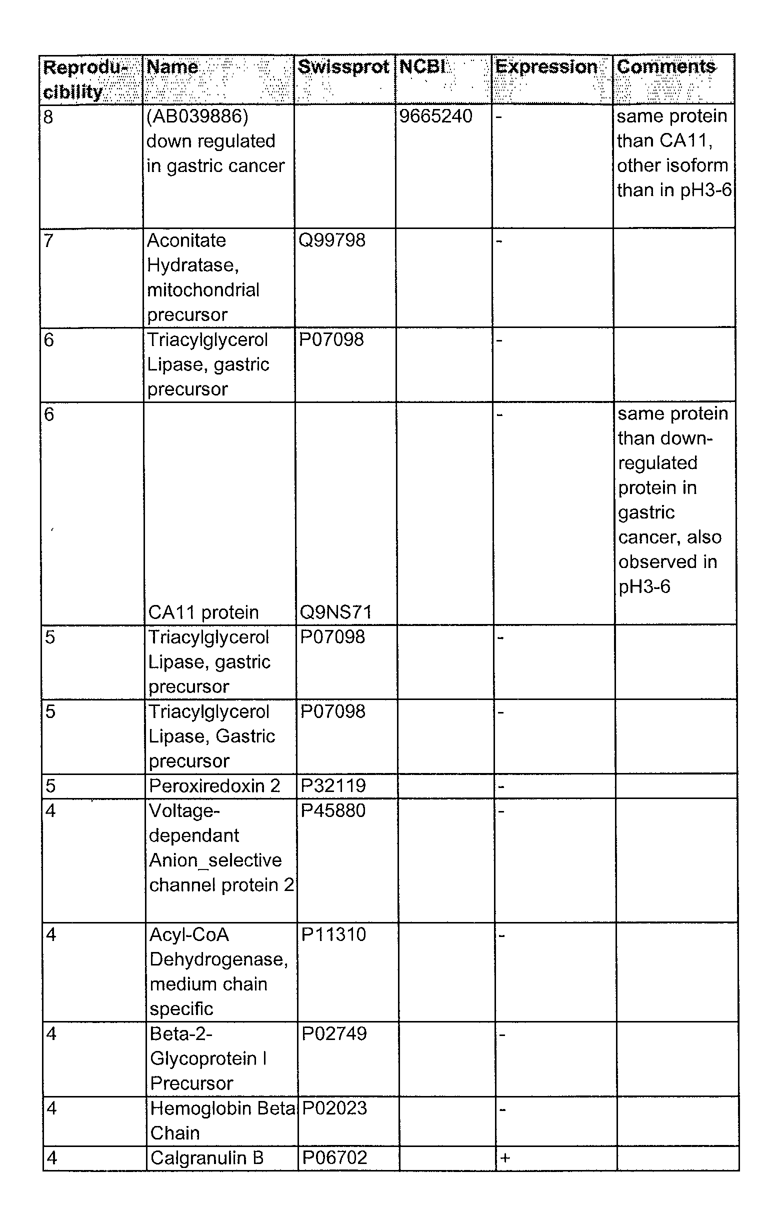

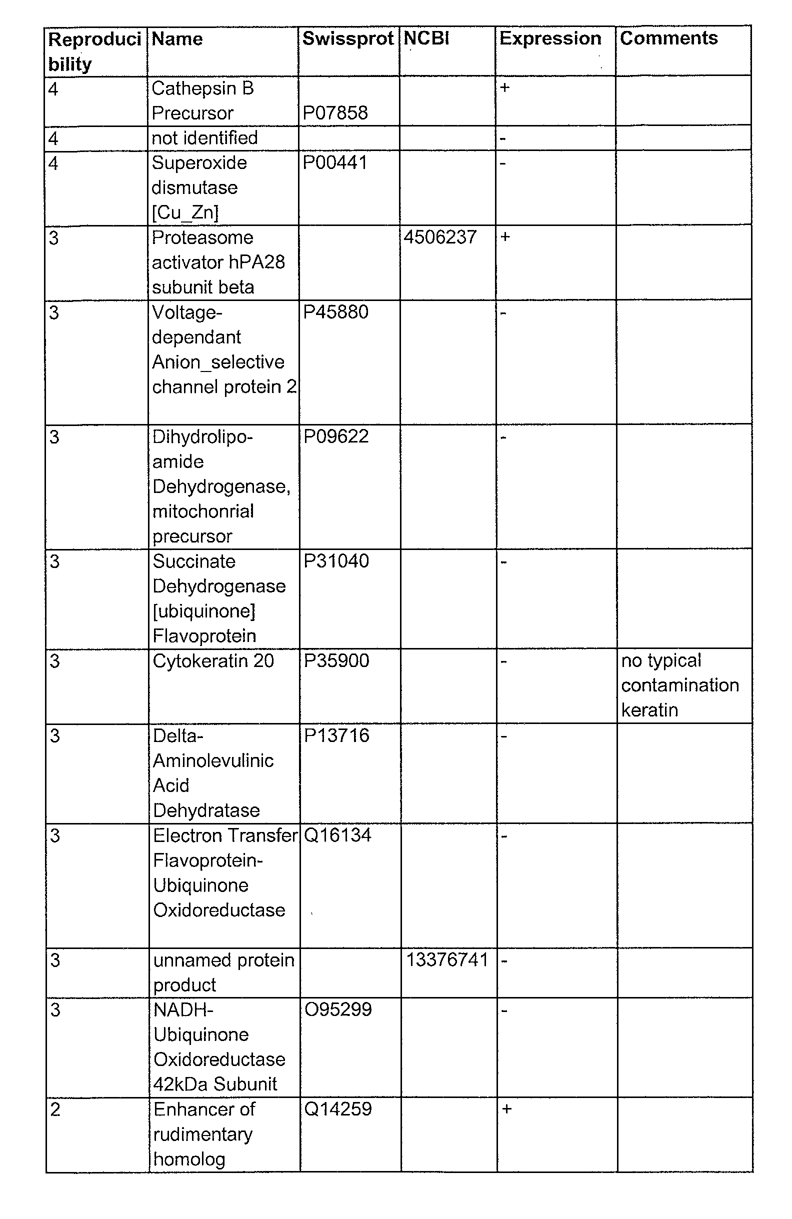

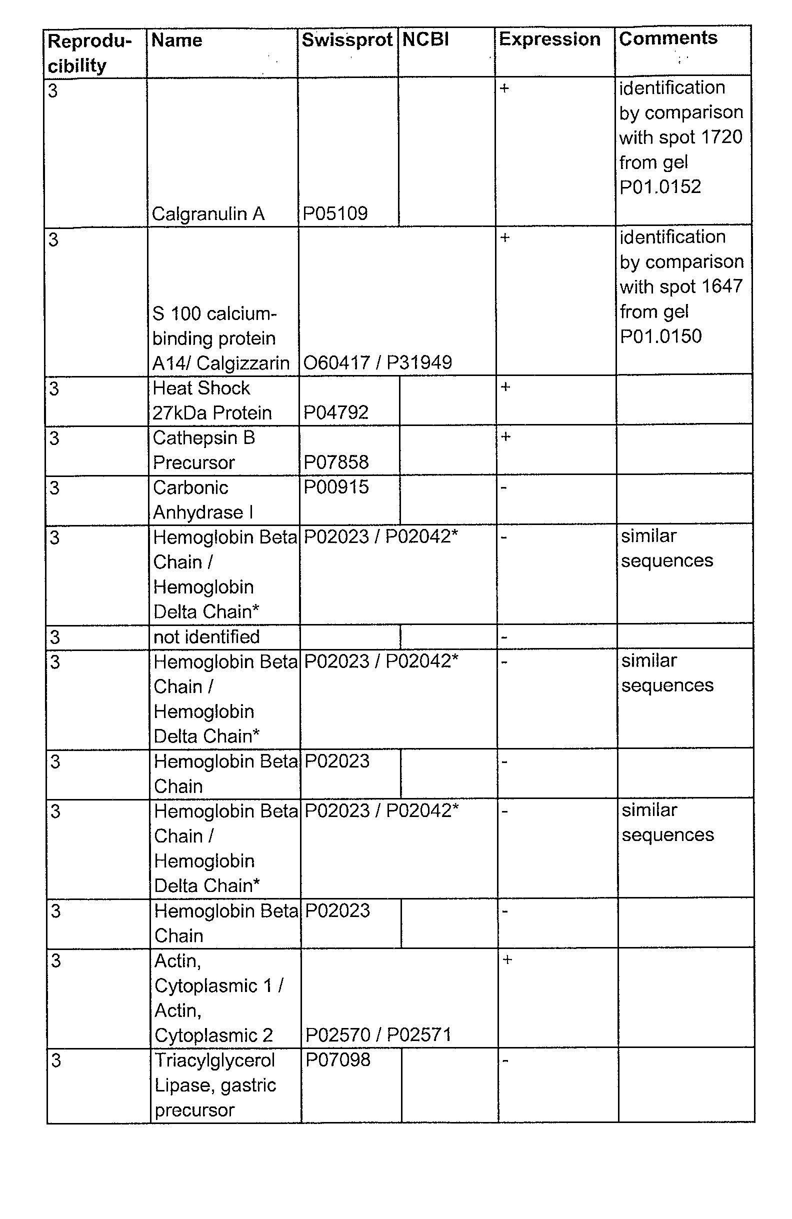

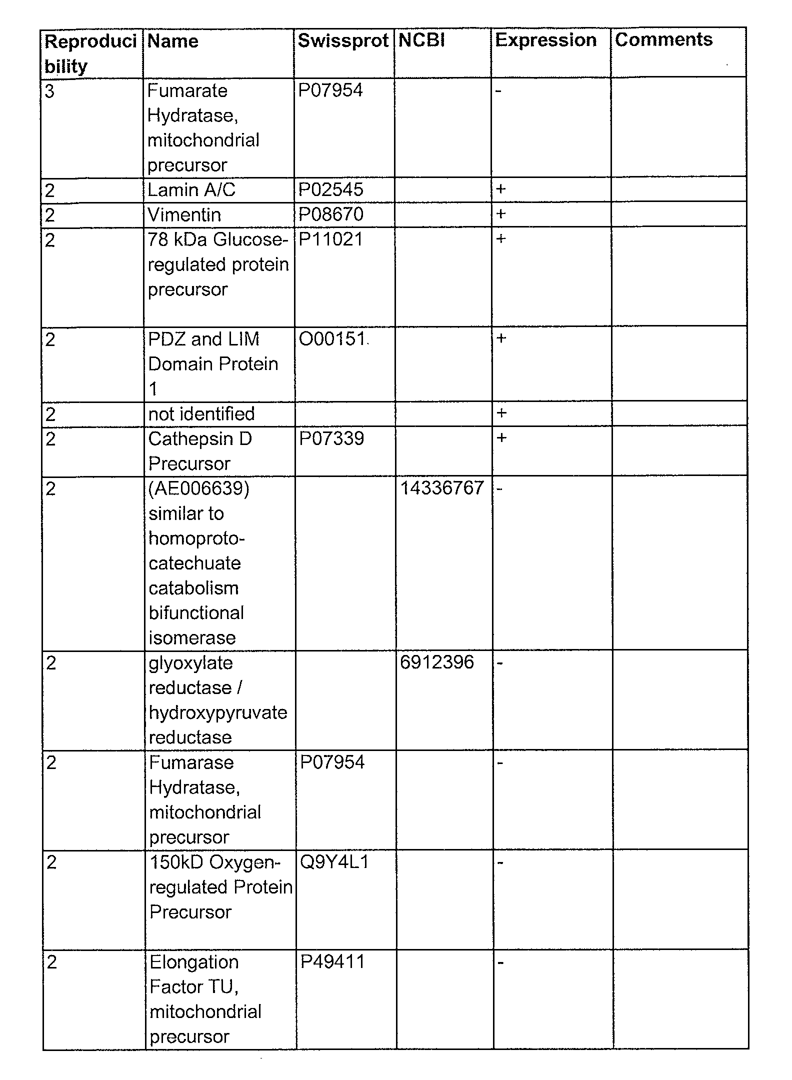

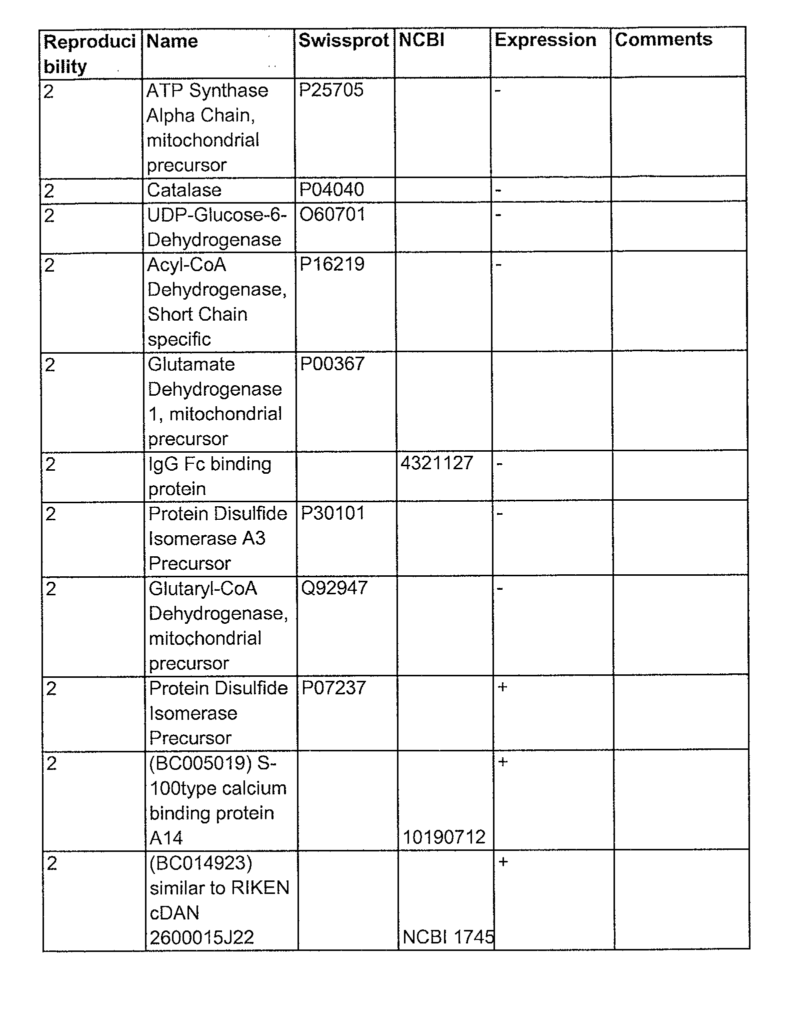

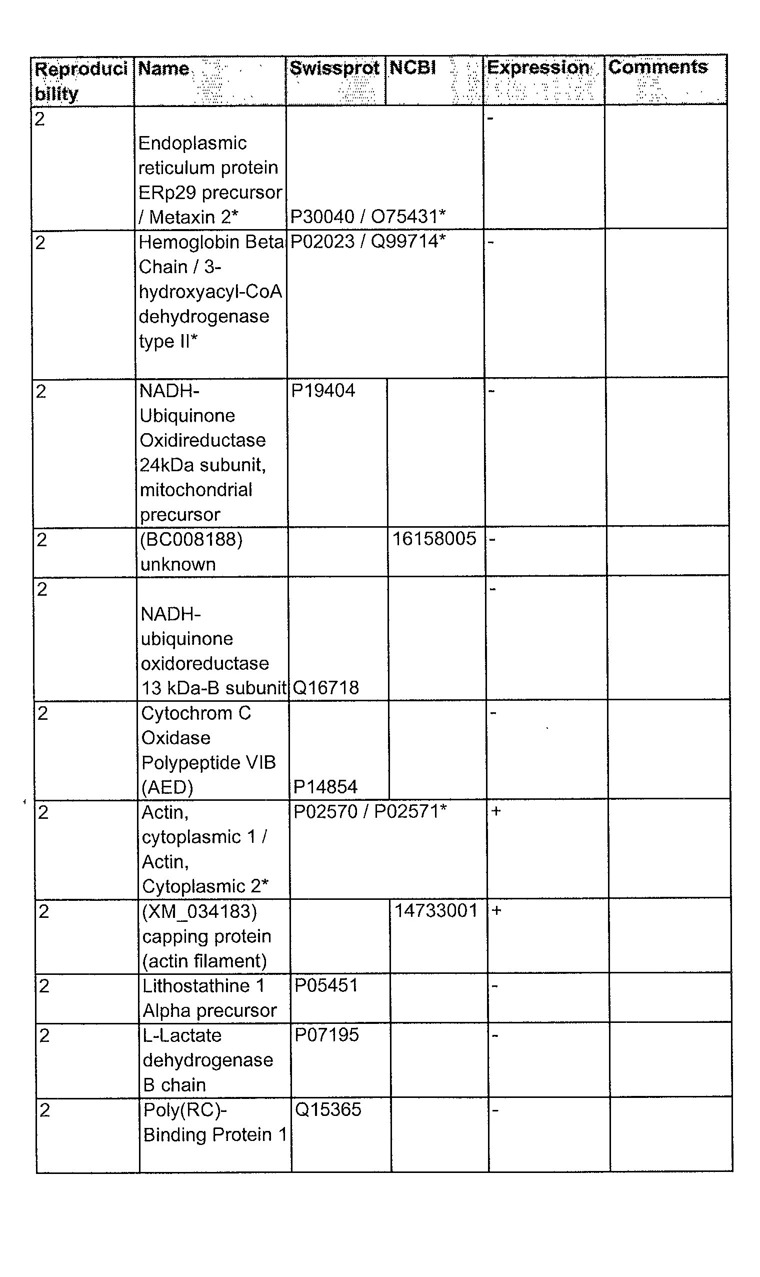

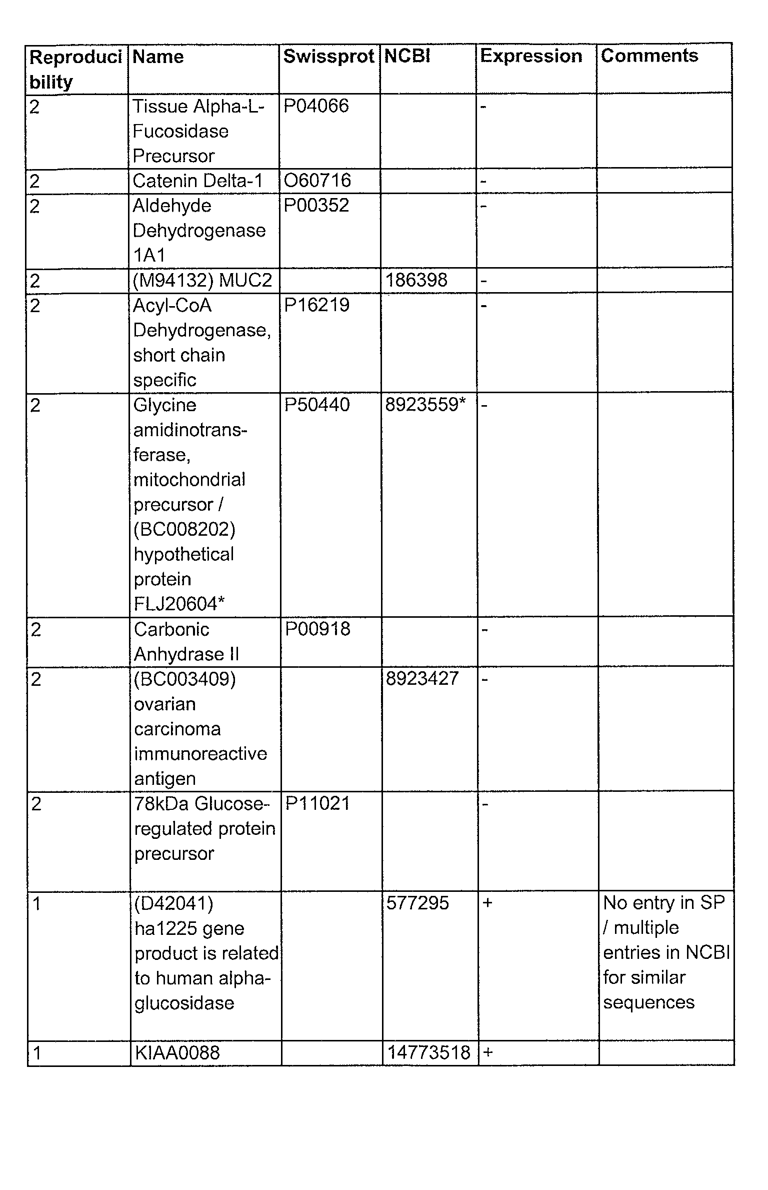

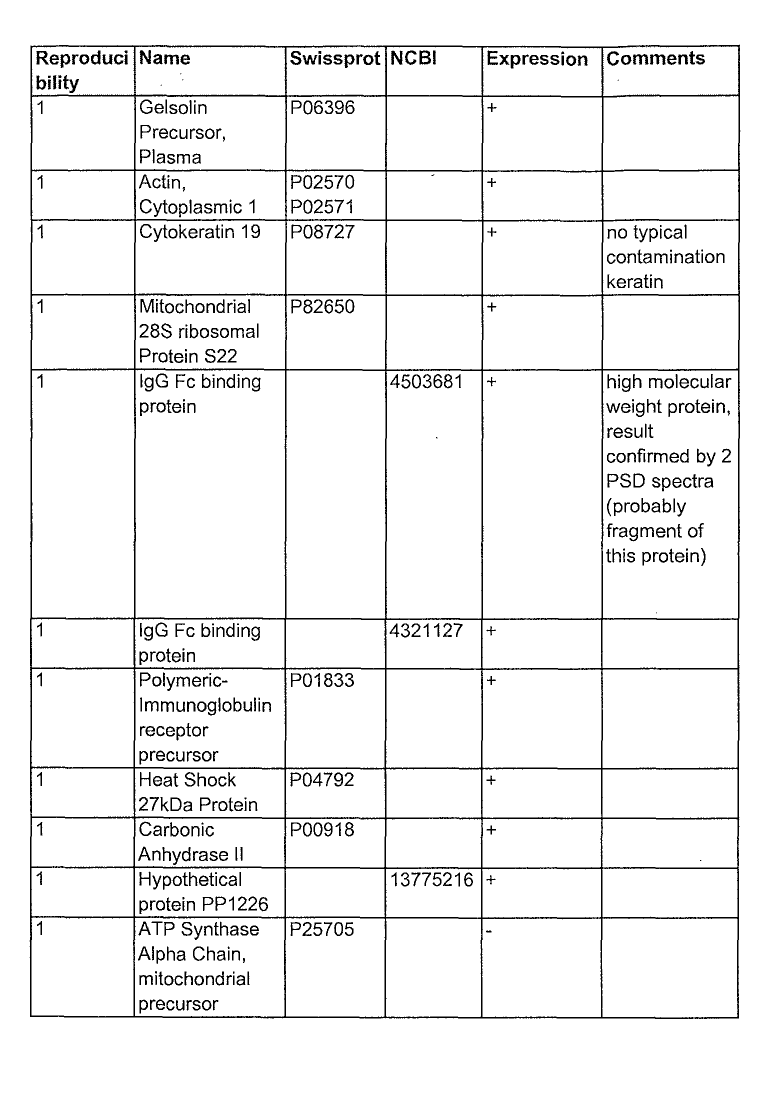

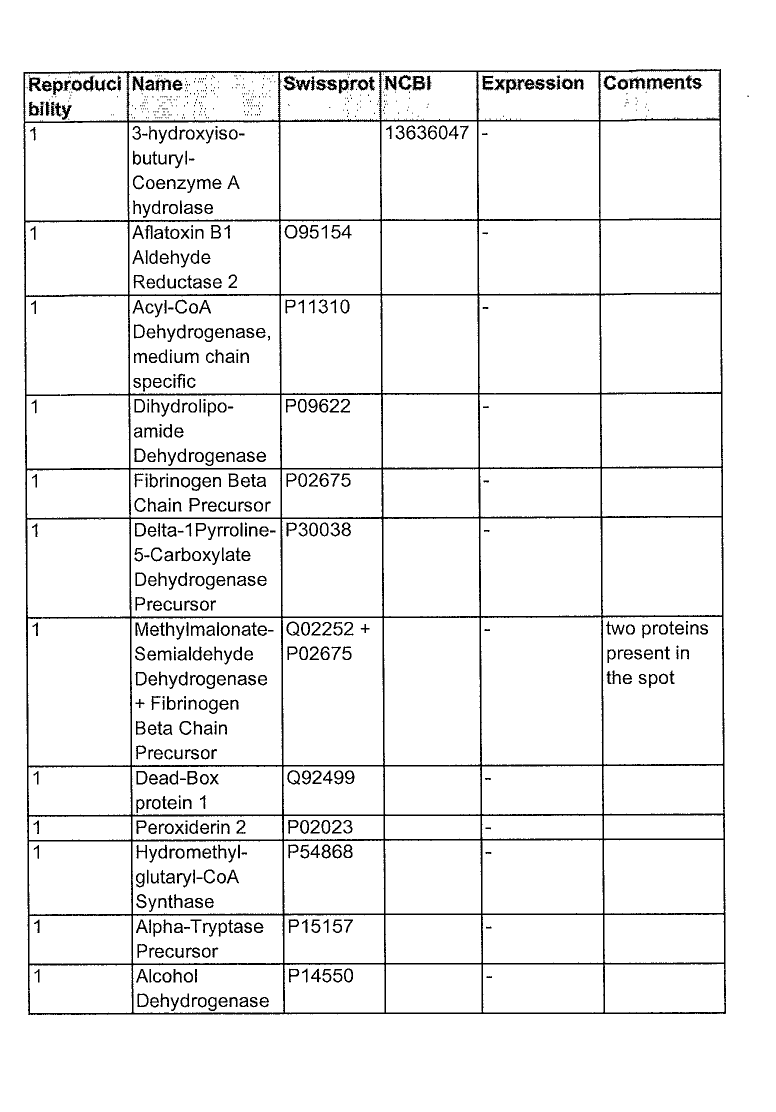

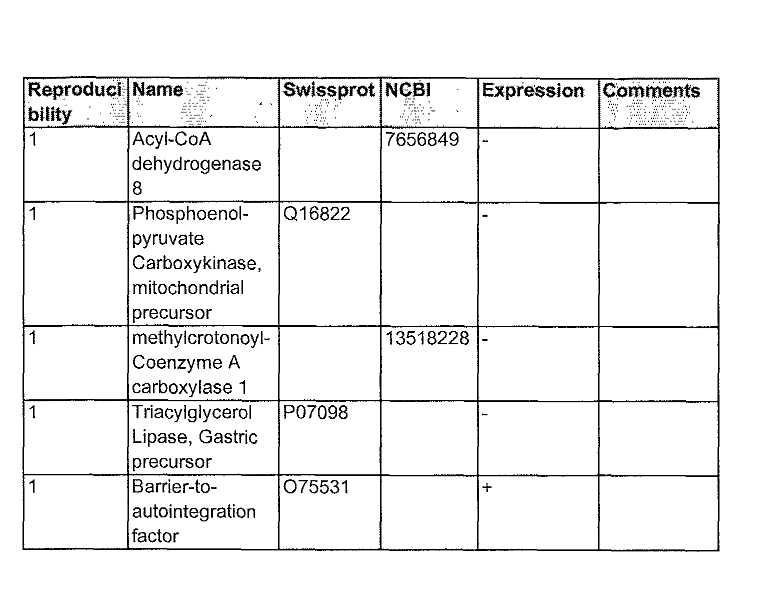

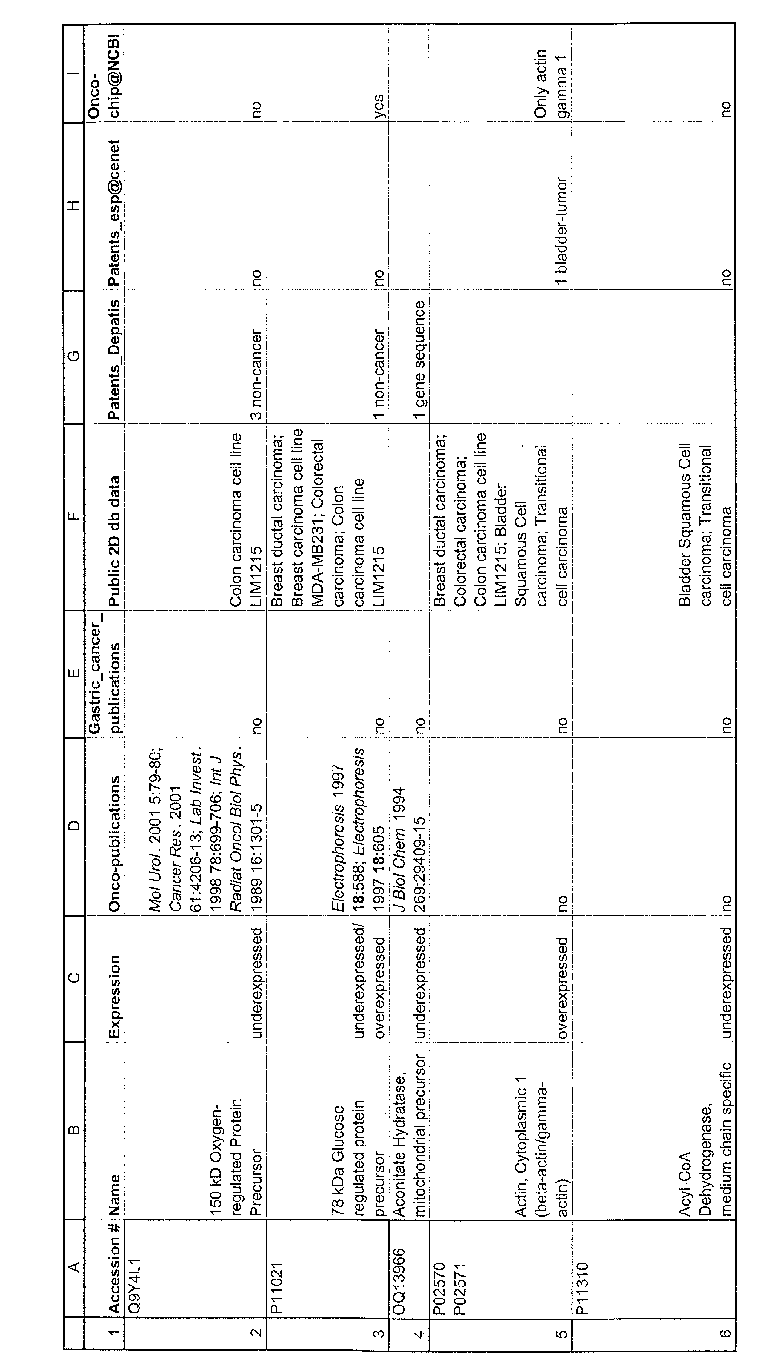

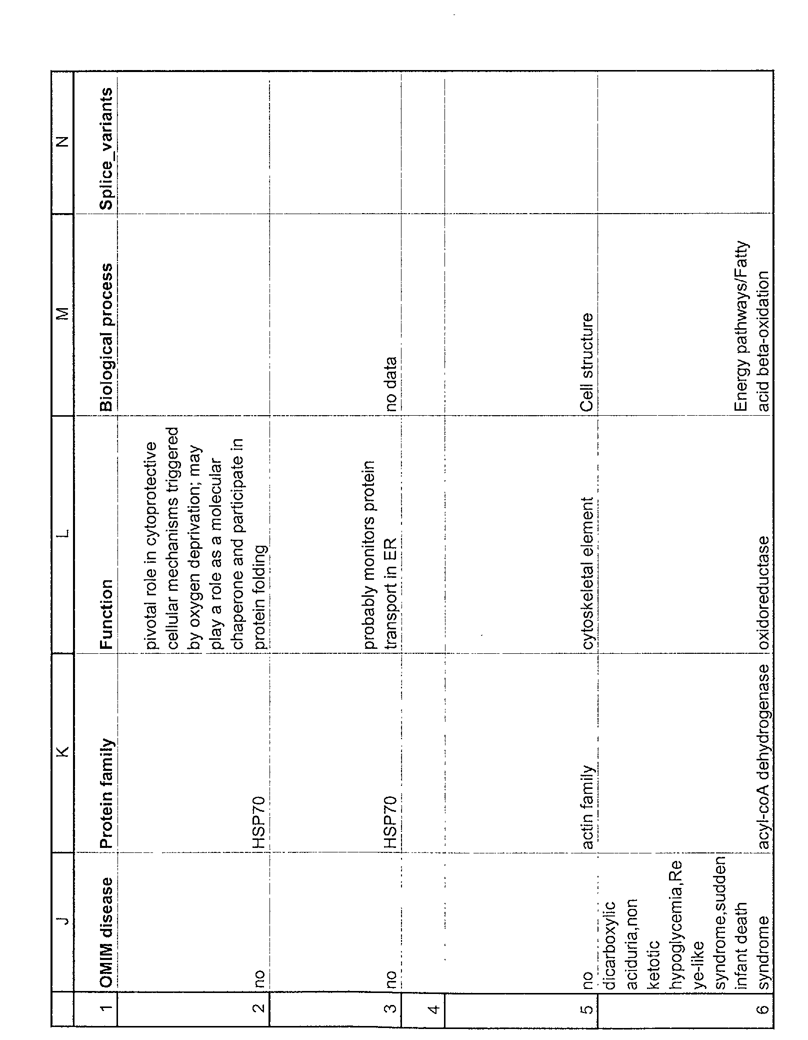

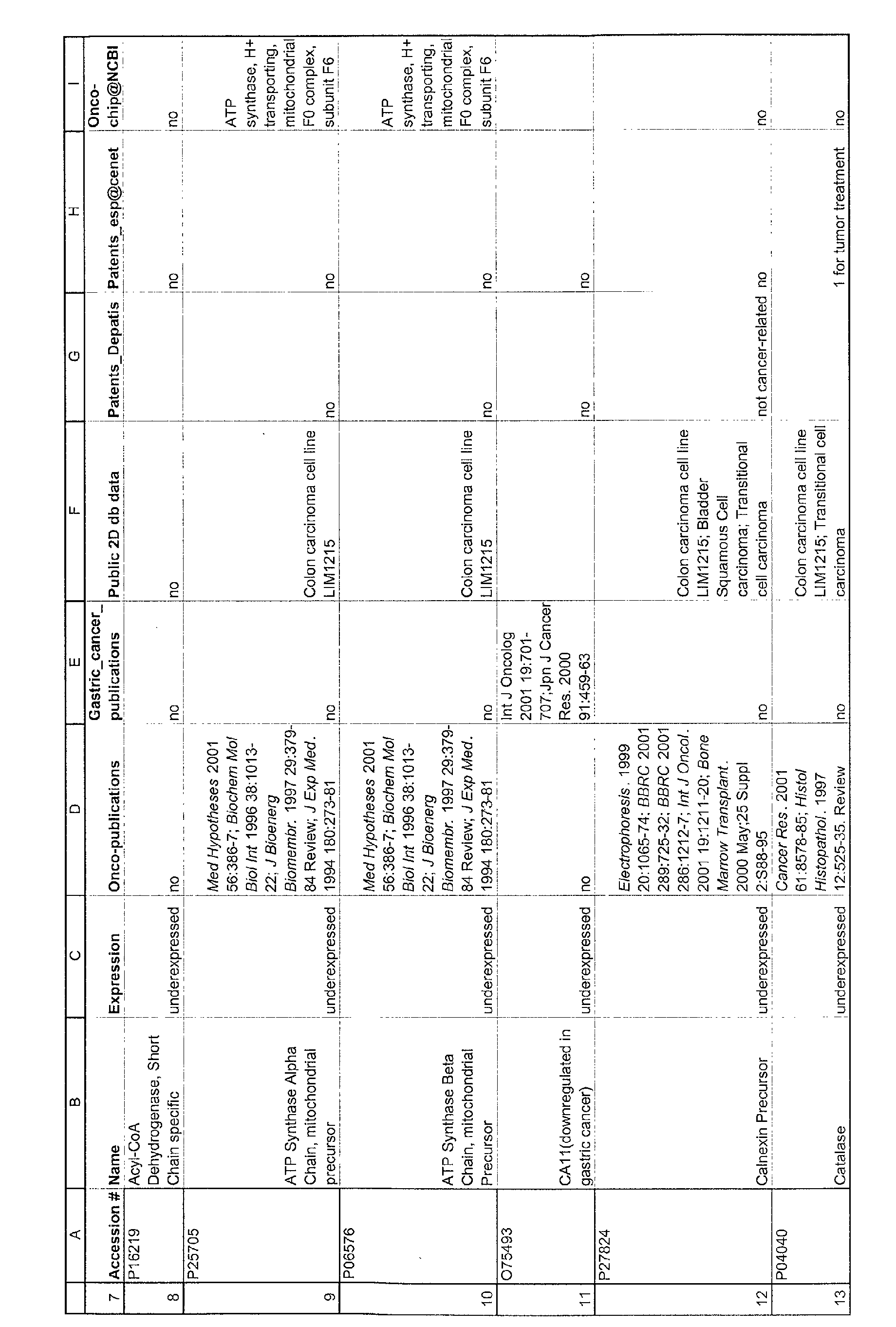

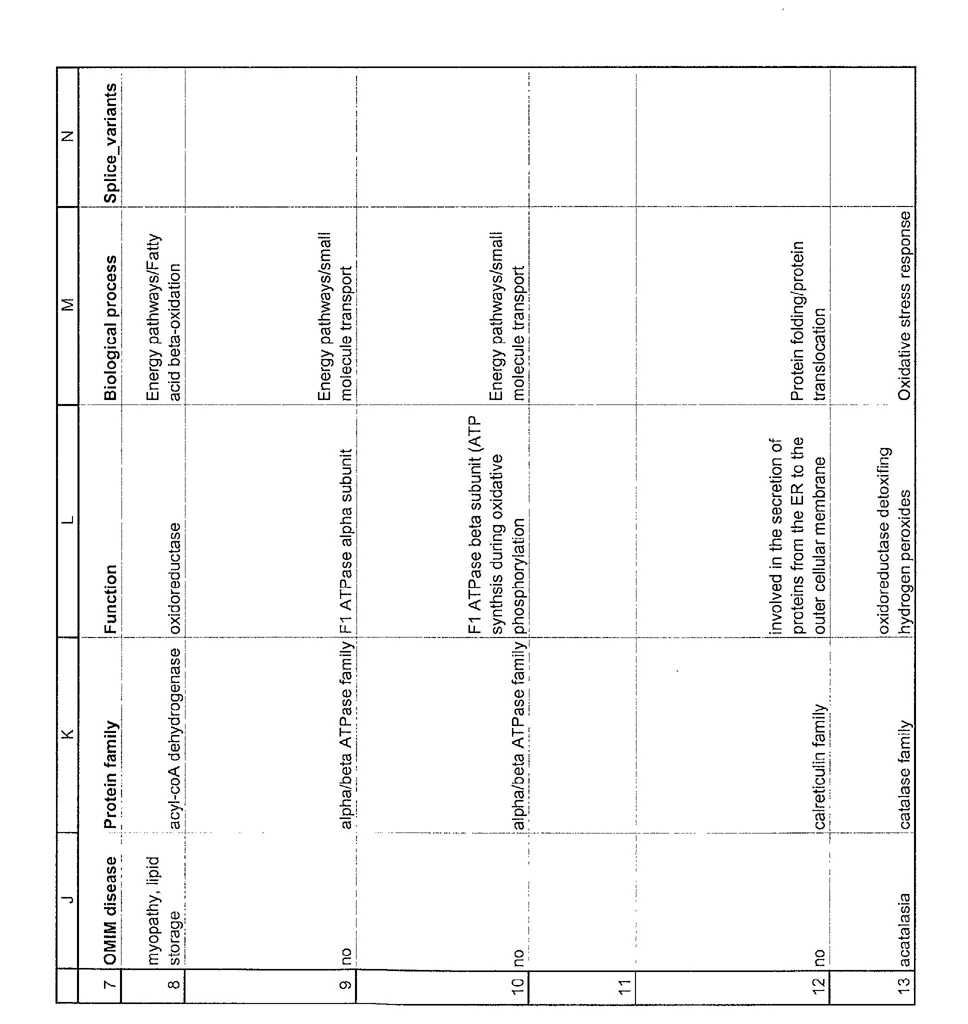

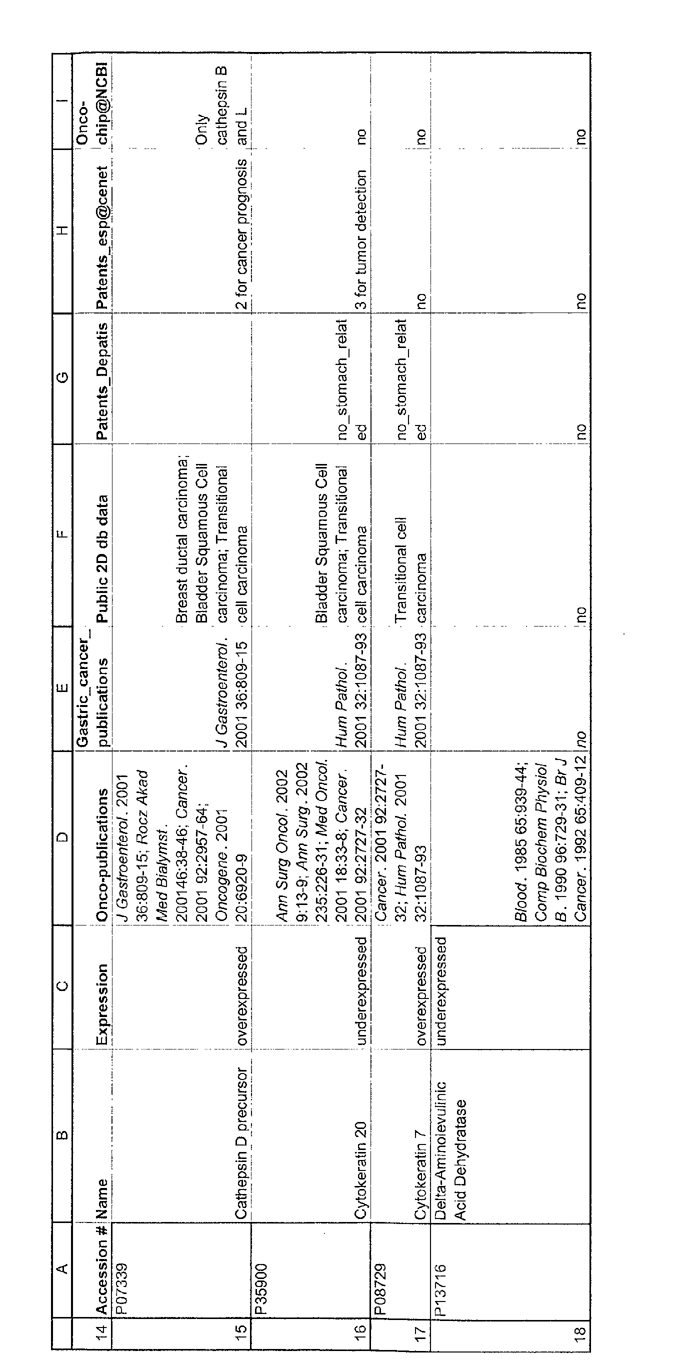

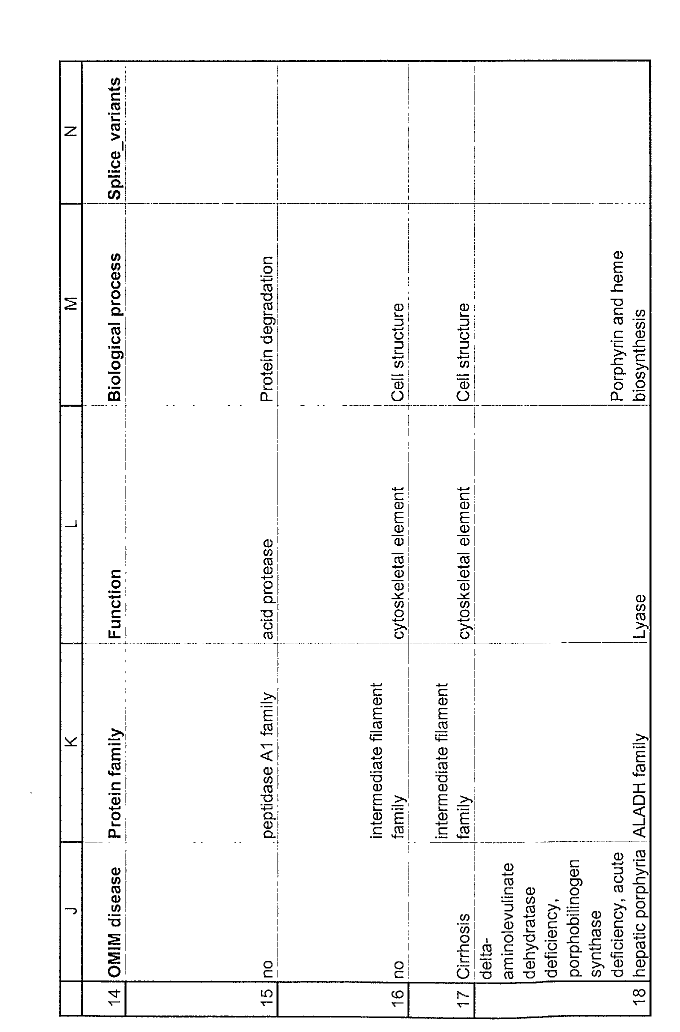

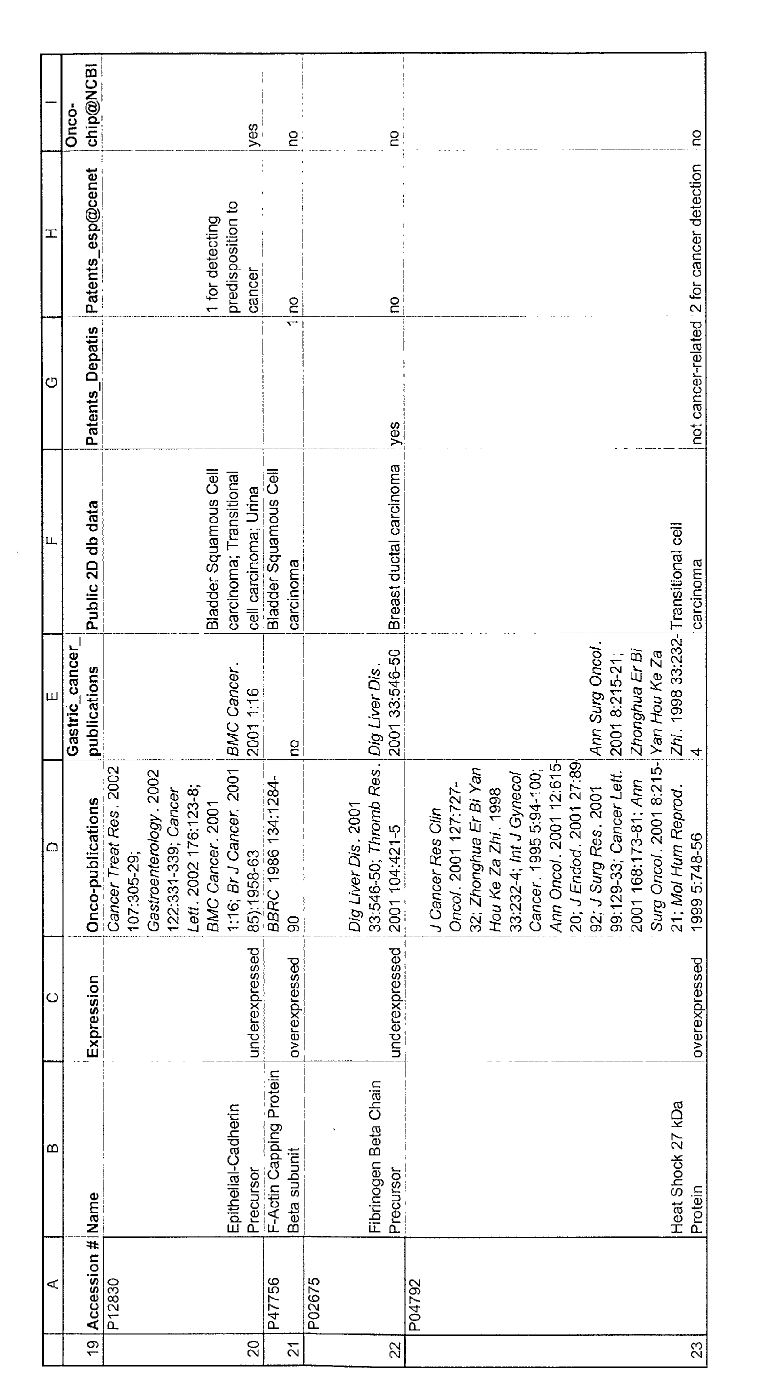

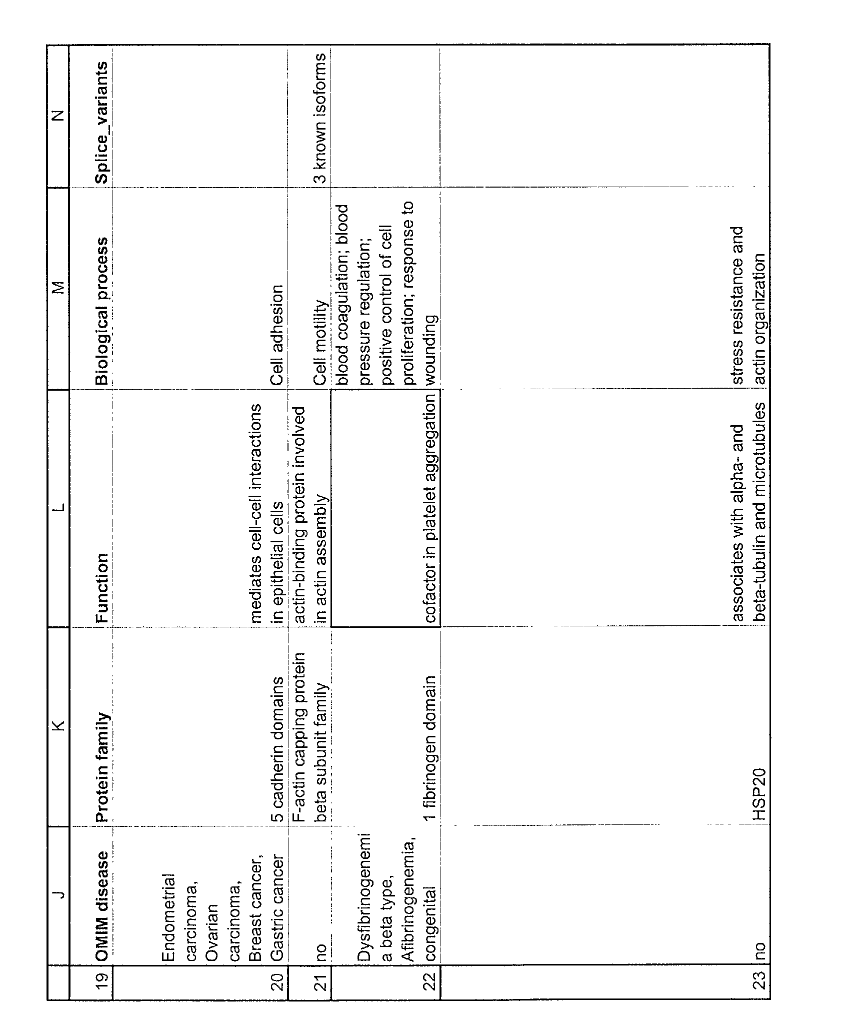

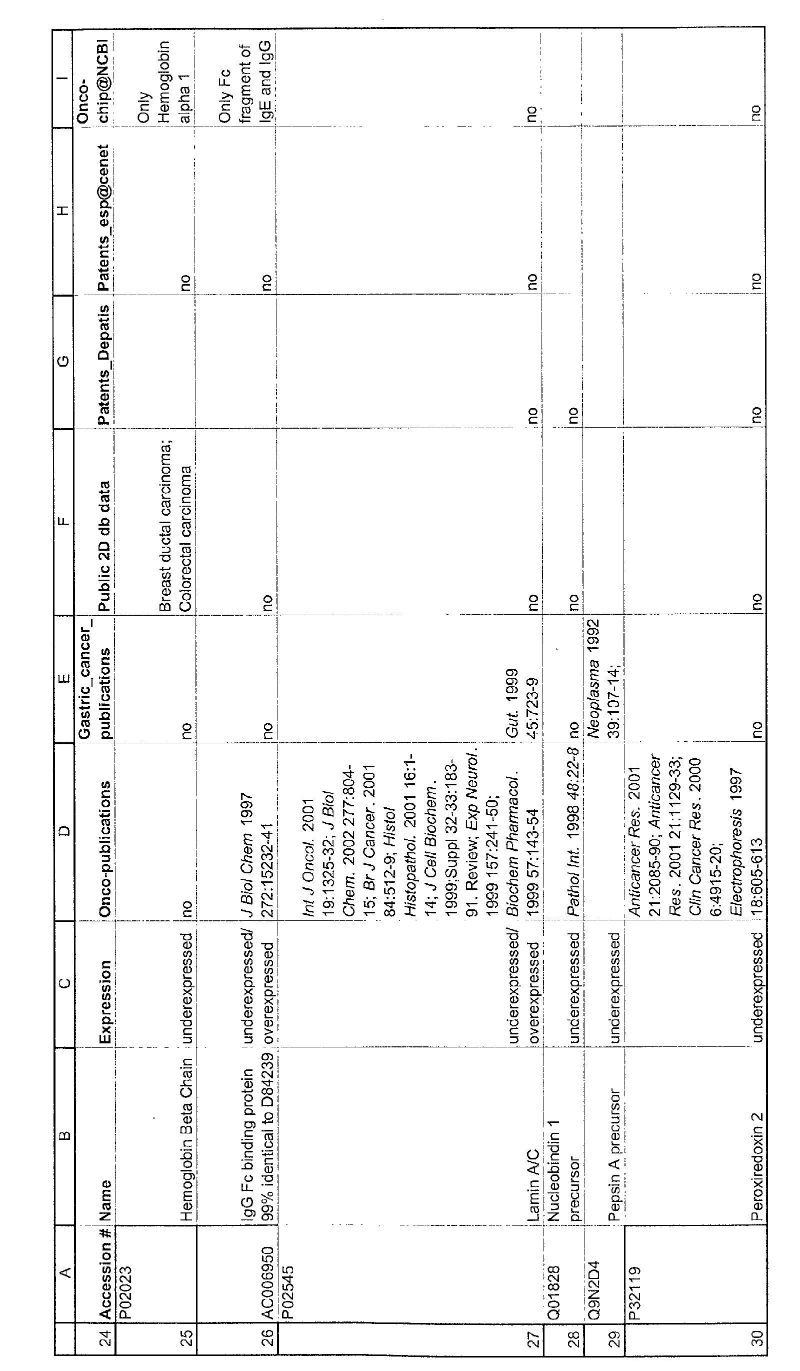

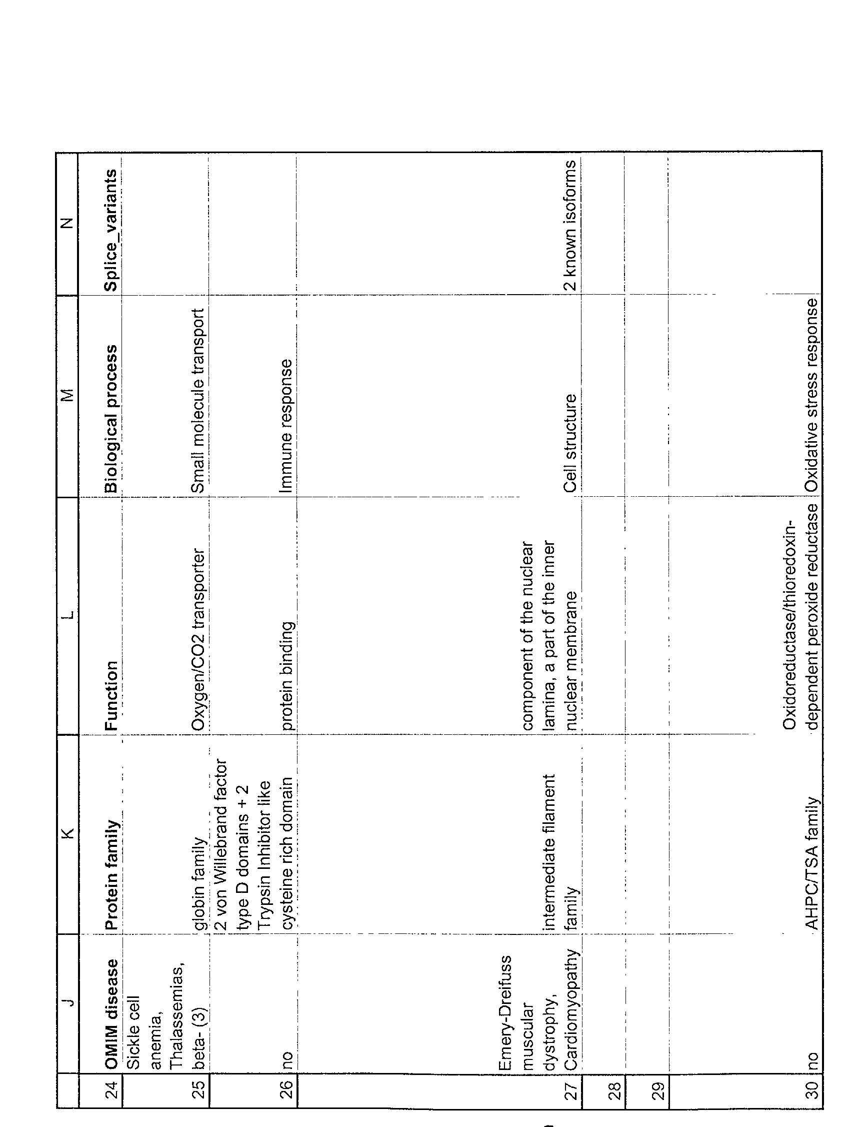

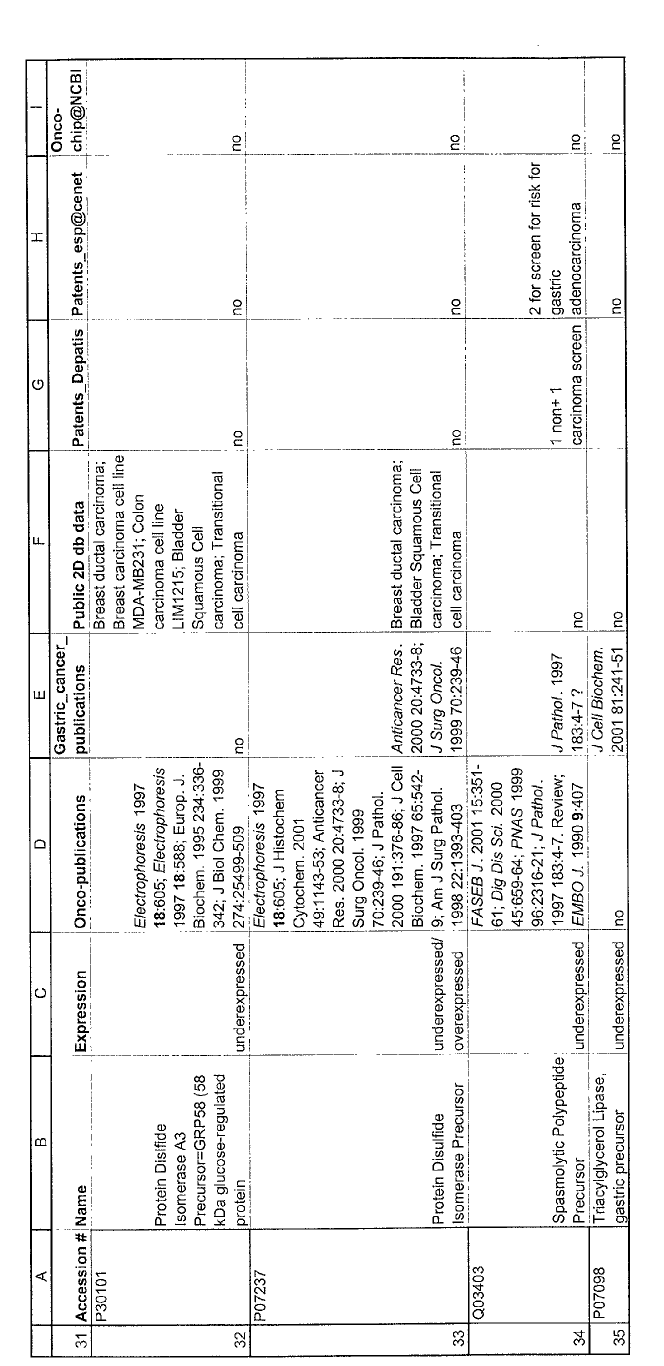

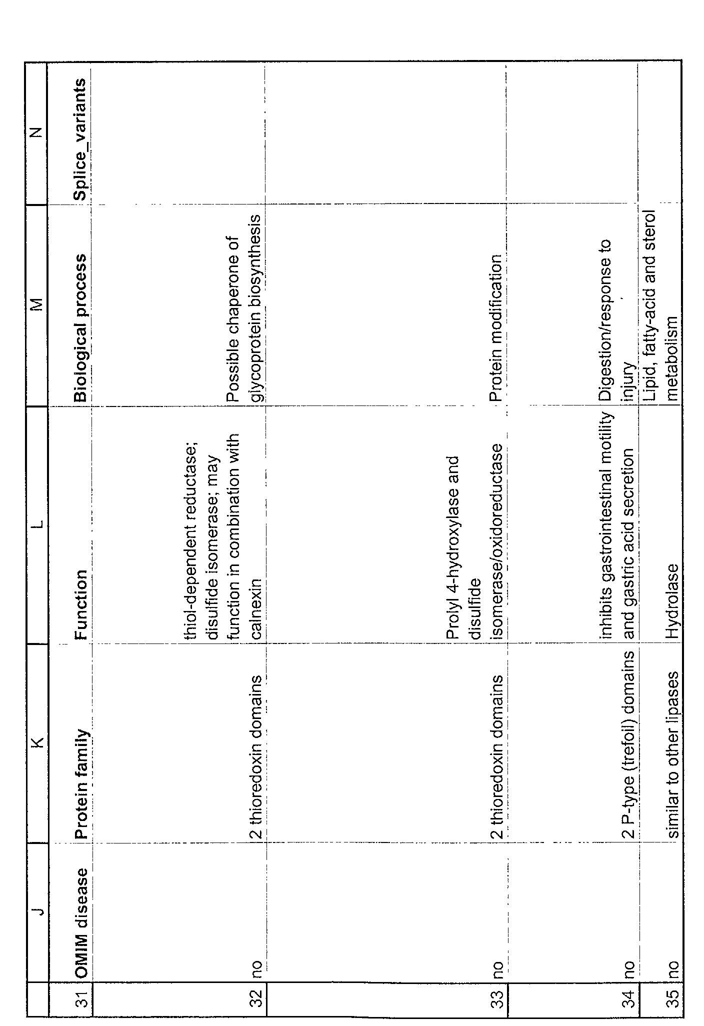

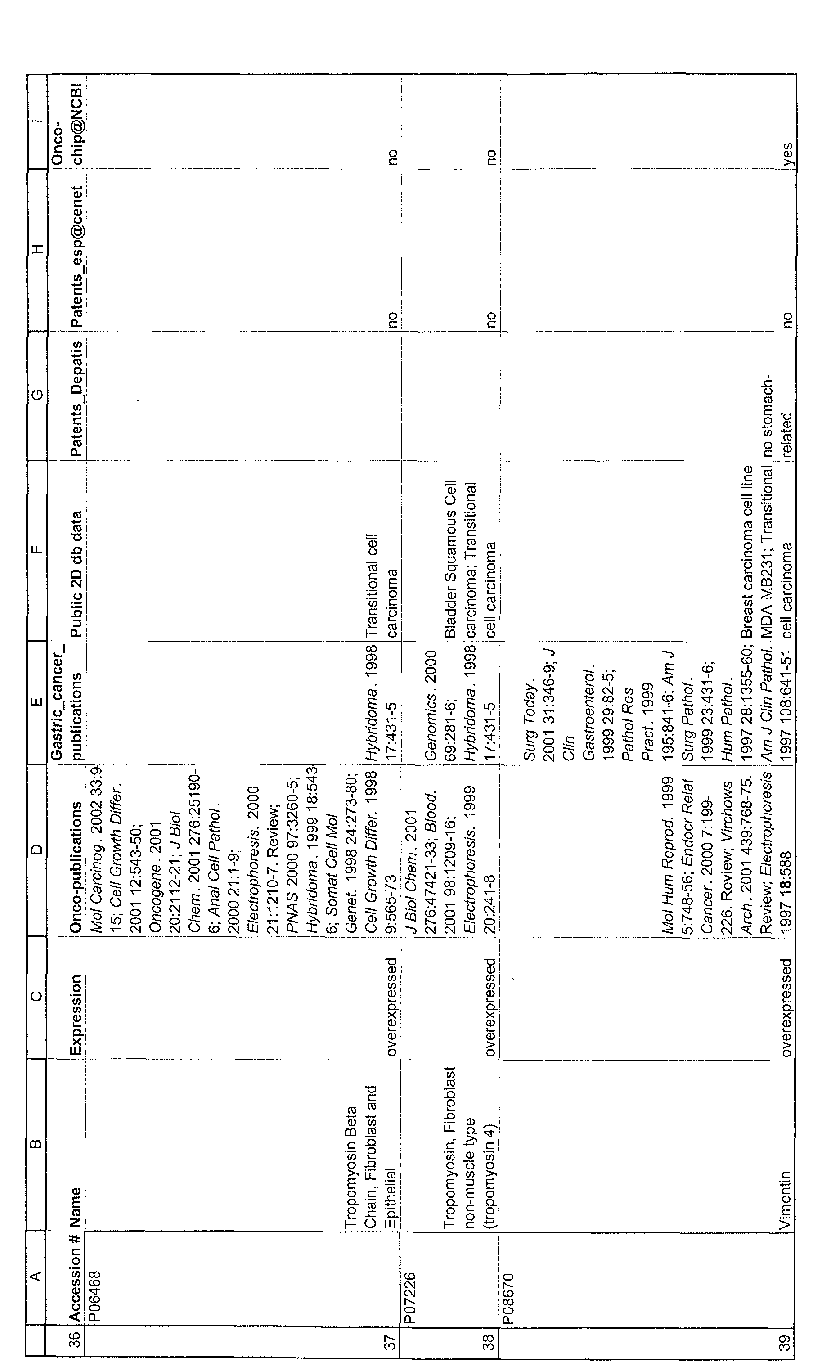

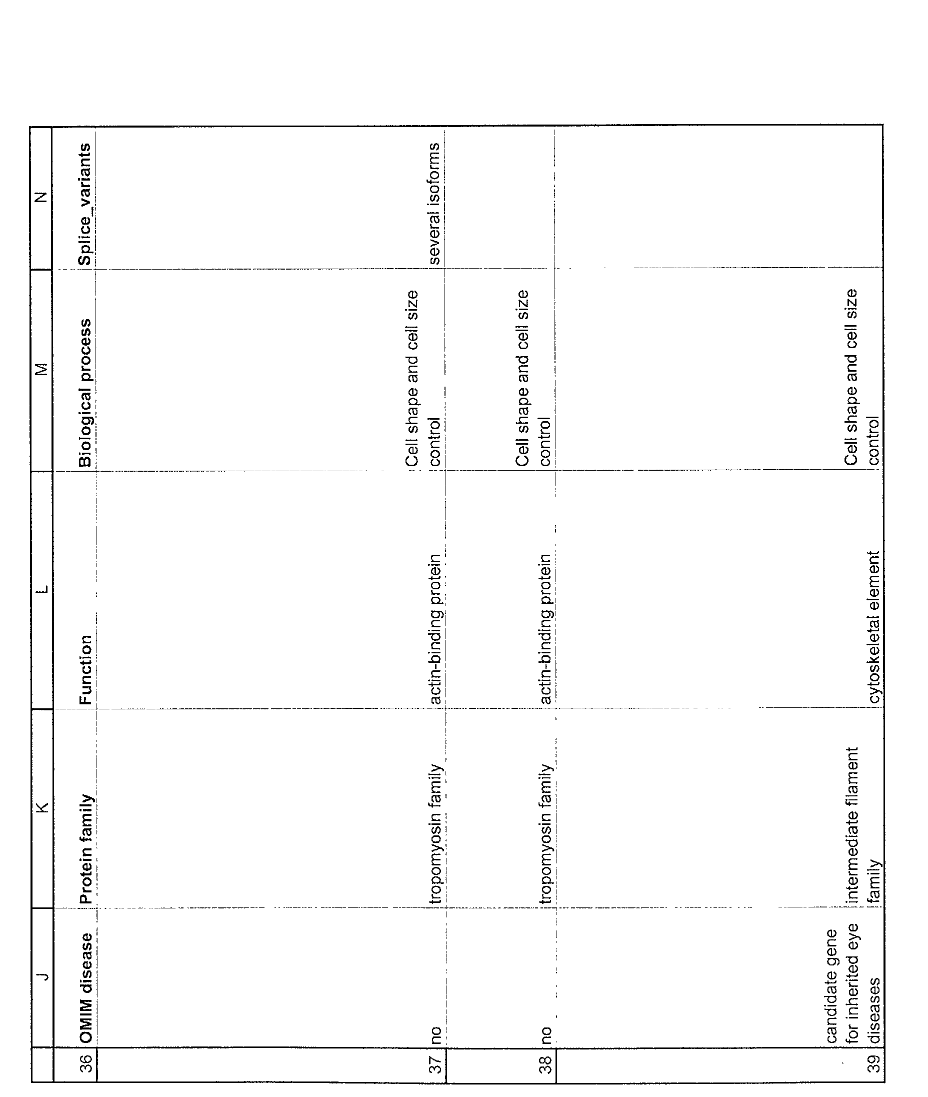

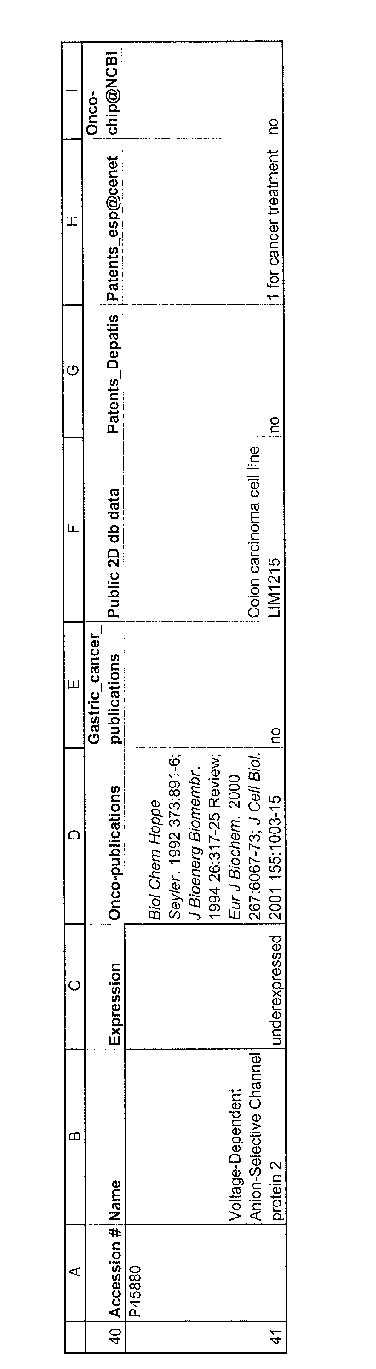

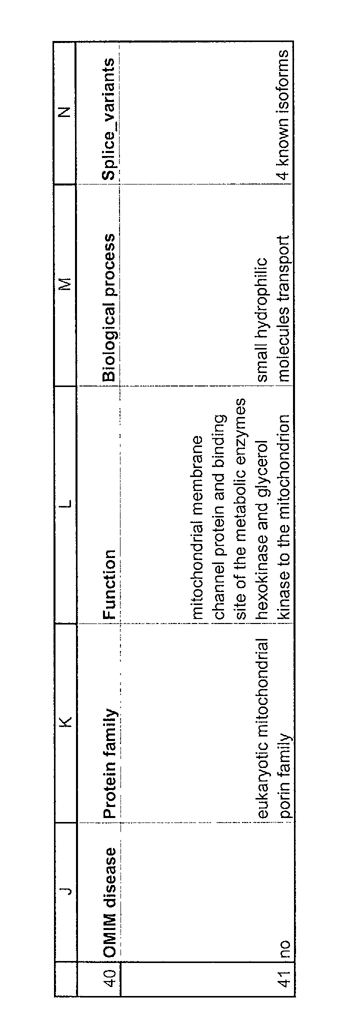

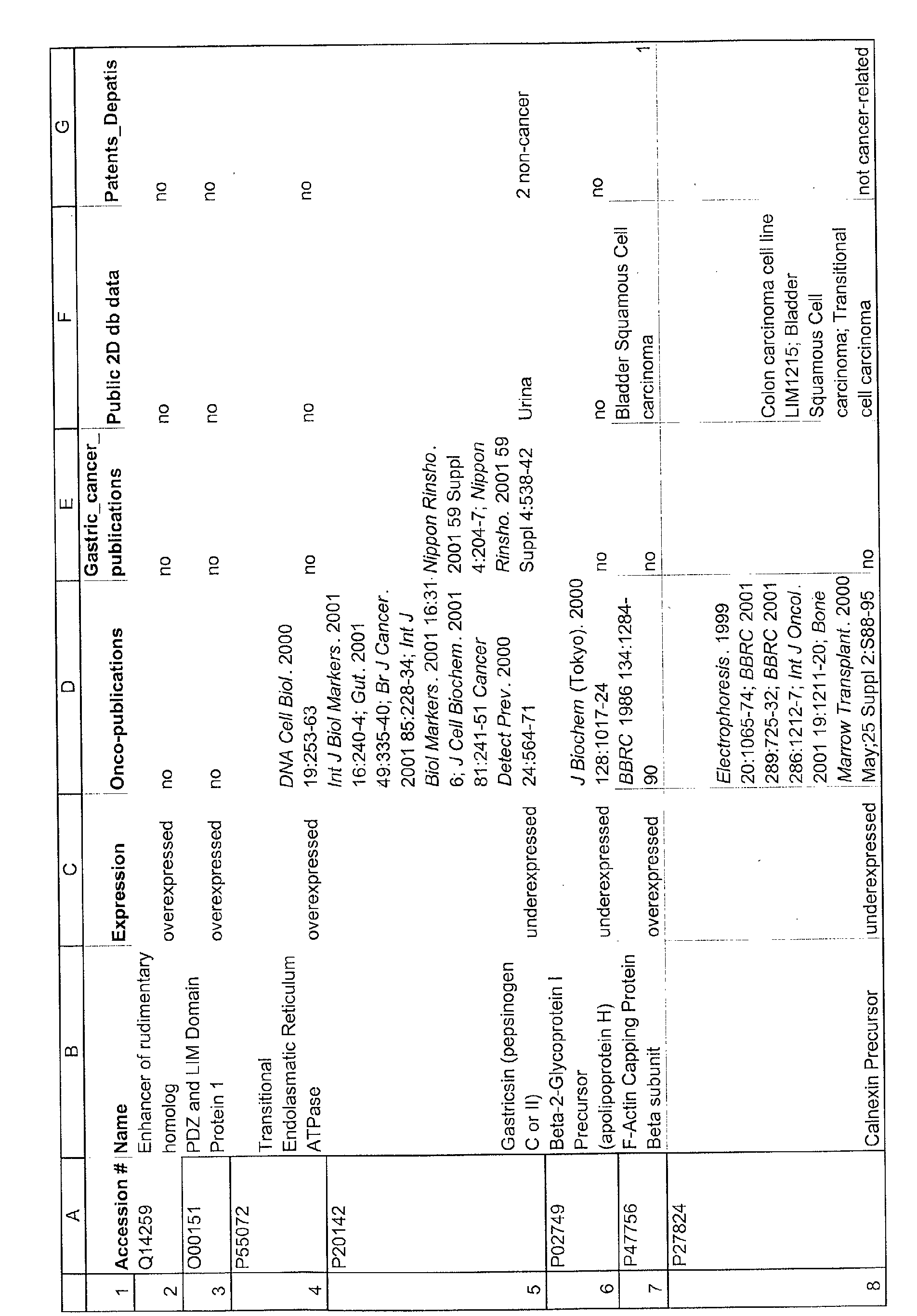

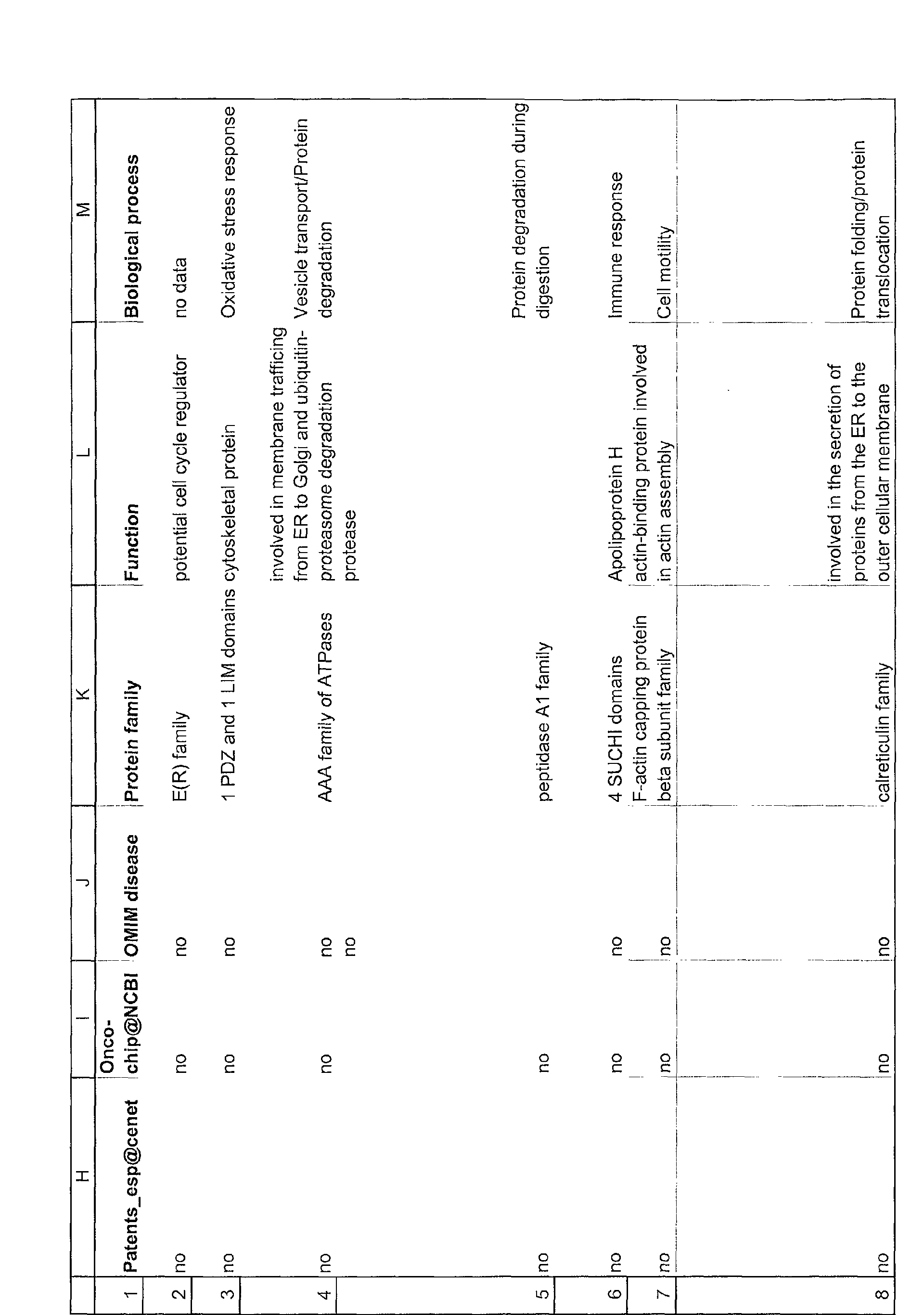

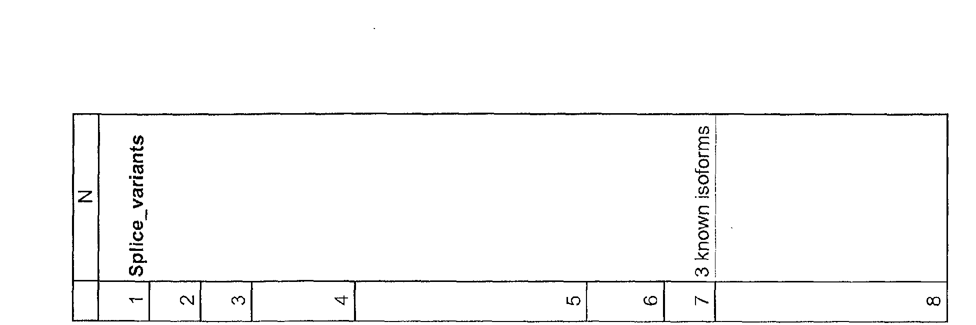

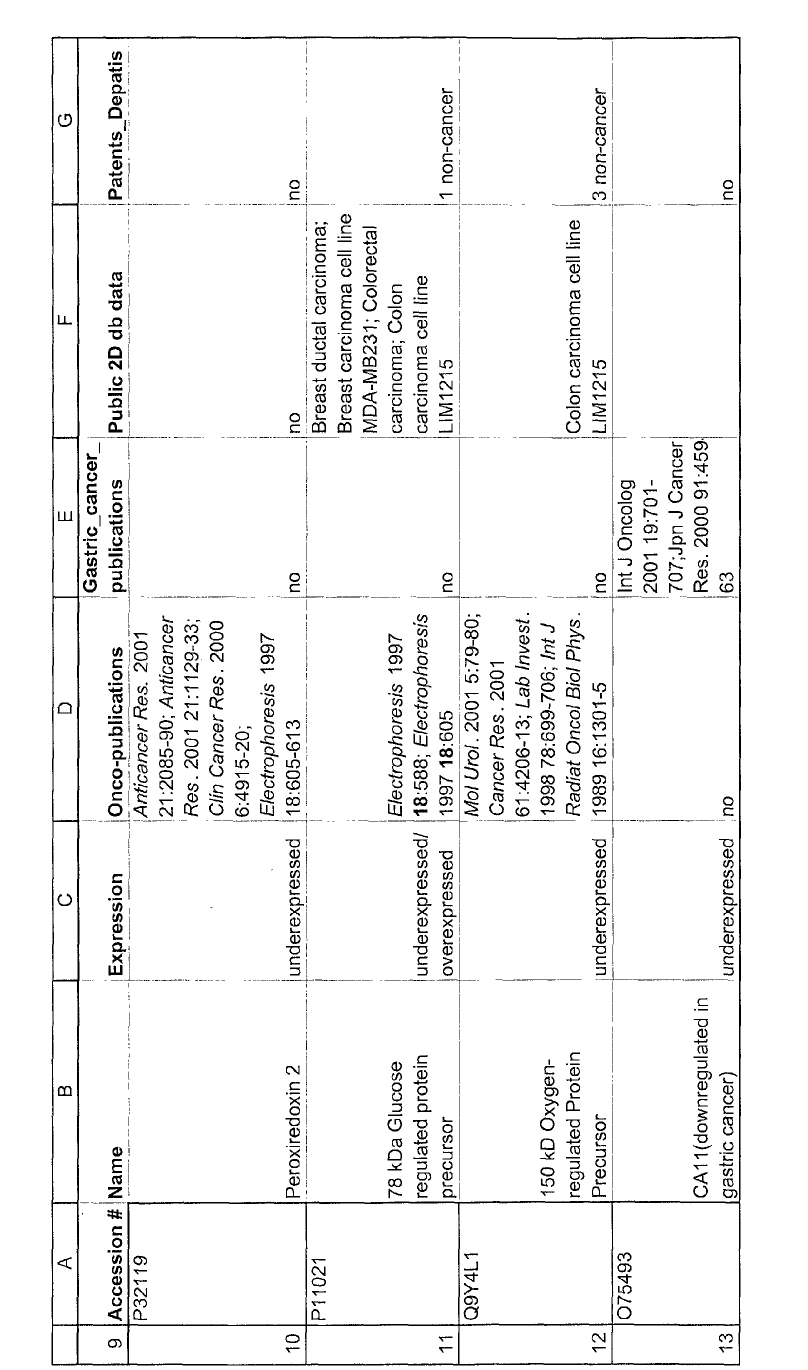

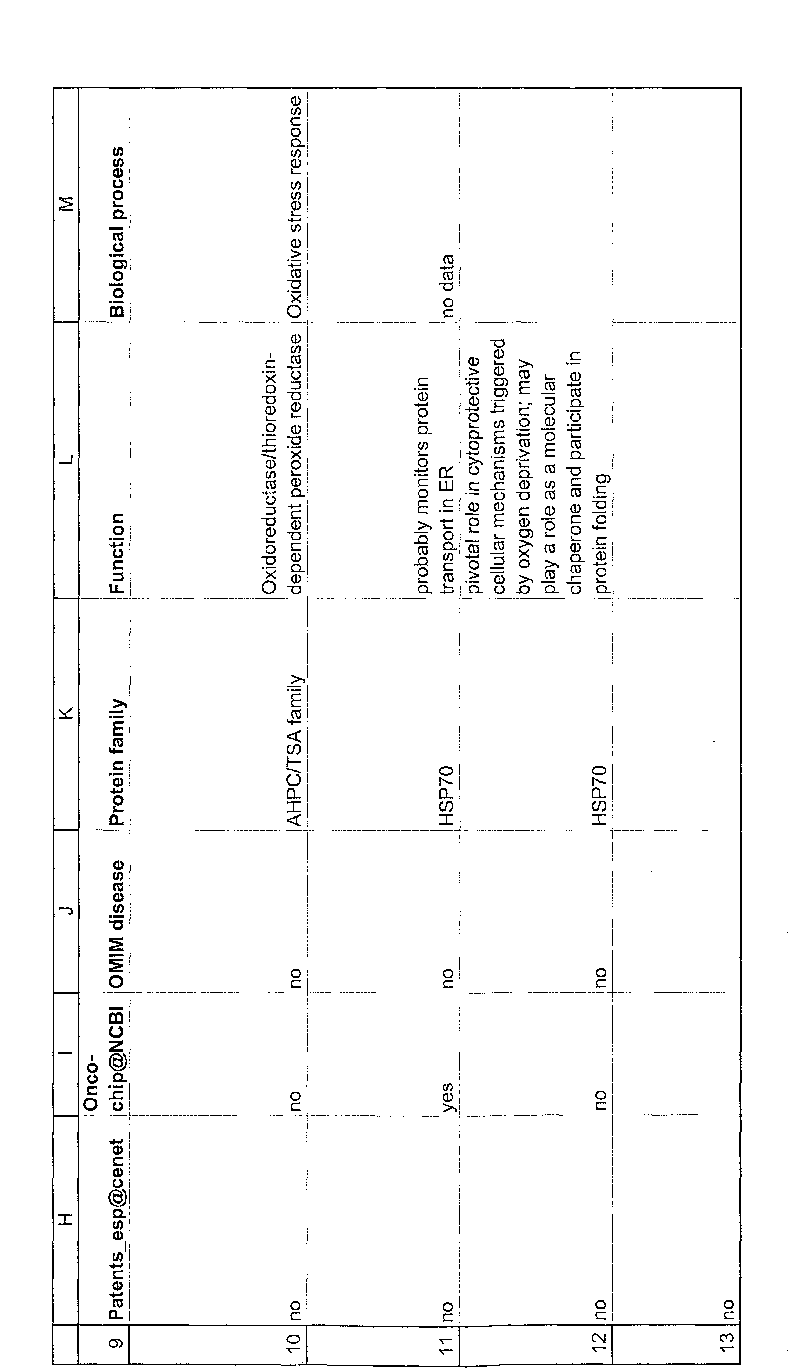

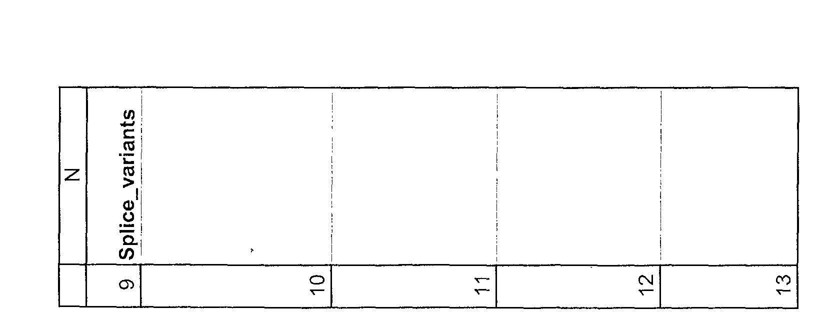

## Claims

1. Use of Q14259 Enhancer of rudimentary homolog, 000151 PDZ and LIM Domain Protein 1, P55072 Transitional Endolasmatic Reticulum ATPase, P20142 Gastricsin, P07492 Beta-2-Glycoprotein I Precursor, P47756 F-Actin Capping Protein Beta subunit, P27824 Calnexin Precursor, P32119 Peroxiredoxin 2, P11021 78 kDa Glucose regulated protein precursor, Q9Y4L1 150 kD Oxygen-regulated Protein Precursor, 075493 CA11, nucleic acid sequences encoding said proteins and/or recognition agents for said proteins or said nucleic acid sequences for the manufacture of an agent for diagnosis, prophylactic or therapeutic treatment of non-steroid dependent cancer.

2. A Method for detecting non-steroid dependent cancer cells in a sample from a patient, wherein the method comprising providing the sample and detecting the level of one or more proteins selected from the group consisting of Q14259 Enhancer of rudimentary homolog, 000151 PDZ and LIM Domain Protein 1, P55072 Transitional Endolasmatic Reticulum ATPase, P20142 Gastricsin, P07492 Beta-2-Glycoprotein I Precursor, P47756 F-Actin Capping Protein Beta subunit, P27824 Calnexin Precursor, P32119 Peroxiredoxin 2, P11021 78 kDa Glucose regulated protein precursor, Q9Y4L1 150 kD Oxygen-regulated Protein Precursor and/or 075493 CA11 in the sample, and comparing the level of the one or more proteins with a control level that is representative of a level in a normal, cancer-free patient, wherein an modified level of the proteins in the sample compared to the level of the proteins in the control sample indicates the presence of non-steroid dependent cancer in the patient.

3. The method of claim 2 wherein the proteins are detected by determining the copy number of nucleic acids encoding for the proteins, the expression level of a gene or genes encoding the protein and/or by measuring the level of the proteins or protein activity in the sample.

4. The method according to claim 2 or 3, wherein the method comprising contacting the sample with a recognition agent for one or more of the proteins or nucleic acid sequences encoding said proteins and detecting binding of the recognition agent to at least one of the proteins or the nucleic acid sequences in the prepared sample as an indication of the presence of cancer cells in the sample.

5. The method of claim 2 to 4, wherein binding of the recognition agent to one or more of the proteins is detected by immunoblotting or immunohistochemical analysis, radioimmunoassay, Western blot analysis or enzyme labelling technique.

6. Method of treatment of non-steroid dependent cancer, **characterised by** a modification of the level of Q14259 Enhancer of rudimentary homolog, 000151 PDZ and LIM Domain Protein 1, P55072 Transitional Endolasmatic Reticulum ATPase, P20142 Gastricsin, P07492 Beta-2-Glycoprotein I Precursor, P47756 F-Actin Capping Protein Beta subunit, P27824 Calnexin Precursor, P32119 Peroxiredoxin 2, P11021 78 kDa Glucose regulated protein precursor, Q9Y4L1 150 kD Oxygen-regulated Protein Precursor, 075493 CA11, nucleic acid sequences encoding said proteins and/or recognition agents for said proteins or said nucleic acid sequences.

7. Method according to claim 6, **characterised by** a reduction of levels of Q14259 Enhancer of rudimentary homolog, 000151 PDZ and LIM Domain Protein 1, P55072 Transitional Endolasmatic Reticulum ATPase, P47756 F-Actin Capping Protein Beta subunit and/or P11021 78 kDa Glucose regulated protein.

8. Method according to claim 7, **characterised by** down-regulation of promoters of Q14259 Enhancer of rudimentary homolog, 000151 PDZ and LIM Domain Protein 1, P55072 Transitional Endolasmatic Reticulum ATPase, P47756 F-Actin Capping Protein Beta subunit and/or P11021 78 kDa Glucose regulated protein.

9. Method according to claim 6, **characterised by** a raising of levels of P20142 Gastricsin, P07492 Beta-2-Glycoprotein I Precursor, P27824 Calnexin Precursor, P32119 Peroxiredoxin 2, P11021 78 kDa Glucose regulated protein precursor, Q9Y4L1 150 kD Oxygen-regulated Protein Precursor and/or 075493 CA11.

10. Method according to claim 9, **characterised by** up-regulation of promoters of P20142 Gastricsin, P07492 Beta-2-Glycoprotein I Precursor, P27824 Calnexin Precursor, P32119 Peroxiredoxin 2, P11021 78 kDa Glucose regulated protein precursor, Q9Y4L1 150 kD Oxygen-regulated Protein Precursor and/or 075493 CA11.

11. A kit for detection of cancer cells in a sample comprising at least one protein selected from the group consisting of Q14259 Enhancer of rudimentary homolog, 000151 PDZ and LIM Domain Protein 1, P55072 Transitional Endolasmatic Reticulum ATPase, P20142 Gastricsin, P07492 Beta-2-Glycoprotein I Precursor, P47756 F-Actin Capping Protein Beta subunit, P27824 Calnexin Precursor, P32119 Peroxiredoxin 2, P11021 78 kDa Glucose regulated protein precursor, Q9Y4L1 150 kD Oxygen-regulated Protein Precursor, 075493 CA11, nucleic acid sequences encoding said proteins and/or recognition agents for said proteins or said nucleic acid sequences.
